(19) Europäisches Patentamt
European Patent Office
Office européen des brevets

(11) EP 4 076 443 B1

(12) **EUROPEAN PATENT SPECIFICATION**

(45) Date of publication and mention of the grant of the patent:
**10.09.2025 Bulletin 2025/37**

(21) Application number: **20902198.9**

(22) Date of filing: **14.12.2020**

(51) International Patent Classification (IPC):
***C07D 471/10*** *(2006.01)* ***A61P 25/24*** *(2006.01)*
***A61P 25/28*** *(2006.01)* ***A61P 31/12*** *(2006.01)*
***A61P 35/00*** *(2006.01)* ***A61P 37/00*** *(2006.01)*
***A61K 31/438*** *(2006.01)* ***A61K 45/06*** *(2006.01)*

(52) Cooperative Patent Classification (CPC):
**A61K 45/06; A61P 25/24; A61P 25/28; A61P 31/12; A61P 35/00; A61P 37/00; C07D 471/10**

(86) International application number:
**PCT/US2020/064750**

(87) International publication number:
**WO 2021/126725 (24.06.2021 Gazette 2021/25)**

(54) **SUBSTITUTED 1,3,8-TRIAZASPIRO[4,5]DECANE-2,4-DIONE COMPOUND AS INDOLEAMINE 2,3-DIOXYGENASE (IDO) AND/OR TRYPTOPHAN 2,3-DIOXYGENASE (TDO) INHIBITORS**

SUBSTITUIERTE 1,3,8-TRIAZASPIRO[4,5]DEKAN-2,4-DIONVERBINDUNG ALS INDIOLAMIN-2,3-DIOXYGENOXYSE-(IDO)- UND/ODER TRYPTOPHAN-2,3-DIOXYGENOXYSE-(TDO)-HEMMER

COMPOSÉ DE 1,3,8-TRIAZASPIRO[4,5] DÉCANE-2,4-DIONE SUBSTITUÉ EN TANT QU'INHIBITEURS DE L'INDOLÉAMINE 2,3-DIOXYGÉNASE (IDO) ET/OU DU TRYPTOPHANE 2,3-DIOXYGÉNASE (TDO)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.12.2019 US 201962949006 P**

(43) Date of publication of application:
**26.10.2022 Bulletin 2022/43**

(73) Proprietors:
- **Merck Sharp & Dohme LLC**
  **Rahway, New Jersey 07065-0907 (US)**
- **Iomet Pharma Ltd.**
  **Edinburgh EH16 4UX (GB)**

(72) Inventors:
- **HAN, Yongxin**
  **Boston, MA 02115-5727 (US)**
- **DENG, Yongqi**
  **Newton, MA 02459 (US)**
- **BENNETT, David, Jonathan**
  **Boston, MA 02115-5727 (US)**
- **COWLEY, Phillip, M.**
  **Livingston EH54 9DU (GB)**
- **WISE, Alan**
  **Eddleston Peeblesshire Peebles EH45 8PW (GB)**

(74) Representative: **Merck Sharp & Dohme LLC**
**120 Moorgate**
**London EC2M 6UR (GB)**

(56) References cited:
**WO-A2-2016/071293**
**US-A1- 2015 353 548**
**US-A1- 2017 107 222**
**US-A1- 2018 354 908**
**US-A1- 2020 172 492**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**Description**

BACKGROUND OF THE INVENTION

Kynurenine (Kyn) pathway is a significant metabolic pathway of tryptophan (Trp). The metabolites of the Kyn pathway are closely correlated with numerous diseases. Two main enzymes, indoleamine-2,3-dioxygenase (IDO) and tryptophan-2,3-dioxygenase (TDO), are believed to regulate the first and rate-limiting step of the Kyn pathway. These enzymes are directly or indirectly involved in various diseases, including inflammatory diseases, cancer, diabetes, and mental disorders.

IDO and TDO are found in various cells, such as tumor, stromal, vascular, and immune cells, especially antigen-presenting cells (APCs), but are not clearly expressed in T cells. Most Trp is catabolized by IDO, which is expressed diffusely in the human body and can be induced by interferon gamma (IFN-γ). For example, IFN-γ activation of mononuclear phagocytes significantly increases IDO1 and flux through the Kyn pathway. IDO has an immunomodulating function in suppressing immune activation cells and promoting immunosuppressive cells, which are related to poor survival in various cancer subjects. Additionally, the Kyn/Trp ratio can be used to evaluate IDO activity. TDO is located in the liver and may only work on Trp compared with the lower substrate specificity of IDO.

It has been observed that HeLa cells co-cultured with peripheral blood lymphocytes (PBLs) acquire an immuno-inhibitory phenotype through up-regulation of IDO activity. A reduction in PBL proliferation upon treatment with interleukin-2 (IL2) was believed to result from IDO released by the tumor cells in response to IFN-γ secretion by the PBLs. This effect was reversed by treatment with 1-methyl-tryptophan (IMT), a specific IDO inhibitor. It was proposed that IDO activity in tumor cells may serve to impair antitumor responses (Logan, et al, 2002, Immunology, 105: 478-87).

Several lines of evidence suggest that IDO is involved in induction of immune tolerance. Studies of mammalian pregnancy, tumor resistance, chronic infections and autoimmune diseases have shown that cells expressing IDO can suppress T-cell responses and promote tolerance. Accelerated Trp catabolism has been observed in diseases and disorders associated with cellular immune activation, such as infection, malignancy, autoimmune diseases and AIDS, as well as during pregnancy. For example, increased levels of IFNs and elevated levels of urinary Trp metabolites have been observed in autoimmune diseases; it has been postulated that systemic or local depletion of Trp occurring in autoimmune diseases may relate to the degeneration and wasting symptoms of these diseases. In support of this hypothesis, high levels of IDO were observed in cells isolated from the synovia of arthritic joints. IFNs are also elevated in human immunodeficiency virus (HIV) subjects and increasing IFN levels are associated with a worsening prognosis. Thus, it was proposed that IDO is induced chronically by HIV infection, and is further increased by opportunistic infections, and that the chronic loss of Trp initiates mechanisms responsible for cachexia, dementia and diarrhea and possibly immunosuppression of AIDS subjects (Brown, et al., 1991, Adv. Exp. Med. Biol, 294: 425-35). To this end, it has recently been shown that IDO inhibition can enhance the levels of virus-specific T cells and, concomitantly, reduce the number of virally-infected macrophages in a mouse model of HIV (Portula et al., 2005, Blood, 106: 2382-90).

IDO is believed to play a role in the immunosuppressive processes that prevent fetal rejection in utero. More than 40 years ago, it was observed that, during pregnancy, the genetically disparate mammalian conceptus survives in spite of what would be predicted by tissue transplantation immunology (Medawar, 1953, Symp. Soc. Exp. Biol. 7: 320-38).

Anatomic separation of mother and fetus and antigenic immaturity of the fetus cannot fully explain fetal allograft survival. Recent attention has focused on immunologic tolerance of the mother. Because IDO is expressed by human syncytiotrophoblast cells and systemic tryptophan concentration falls during normal pregnancy, it was hypothesized that IDO expression at the maternal-fetal interface is necessary to prevent immunologic rejection of the fetal allografts. To test this hypothesis, pregnant mice (carrying syngeneic or allogeneic fetuses) were exposed to IMT, and a rapid, T cell-induced rejection of all allogeneic conception was observed. Thus, by catabolizing tryptophan, the mammalian conceptus appears to suppress T-cell activity and defends itself against rejection, and blocking tryptophan catabolism during murine pregnancy allows maternal T cells to provoke fetal allograft rejection (Moan, et al., 1998, Science, 281: 1191-3).

Further evidence for a tumoral immune resistance mechanism based on tryptophan degradation by IDO comes from the observation that most human tumors constitutively express IDO, and that expression of IDO by immunogenic mouse tumor cells prevents their rejection by preimmunized mice. This effect is accompanied by a lack of accumulation of specific T cells at the tumor site and can be partly reverted by systemic treatment of mice with an inhibitor of IDO, in the absence of noticeable toxicity. Thus, it was suggested that the efficacy of therapeutic vaccination of cancer subjects might be improved by concomitant administration of an IDO inhibitor (Uyttenhove et al. , 2003, Nature Med., 9: 1269-74). It has also been shown that the IDO inhibitor, 1-MT, can synergize with chemotherapeutic agents to reduce tumor growth in mice, suggesting that IDO inhibition may also enhance the anti-tumor activity of conventional cytotoxic therapies (Muller et al, 2005, Nature Med., 11: 312-9).

One mechanism contributing to immunologic unresponsiveness toward tumors may be presentation of tumor antigens by tolerogenic host APCs. A subset of human IDO-expressing antigen-presenting cells (APCs) that coexpressed CD 123 (IL3RA) and CCR6 and inhibited T-cell proliferation have also been described. Both mature and immature CD123-positive dendritic cells suppressed T-cell activity, and this IDO suppressive activity was blocked by 1-MT (Munn, et al, 2002, Science, 297: 1867-70). It has also been demonstrated that mouse tumor-draining lymph nodes (TDLNs) contain a subset of plasmacytoid dendritic cells (pDCs) that constitutively express immunosuppressive levels of IDO. Despite comprising only 0.5% of lymph node cells, in vitro, these pDCs potently suppressed T cell responses to antigens presented by the pDCs themselves and also, in a dominant fashion, suppressed T cell responses to third-party antigens presented by nonsuppressive APCs. Within the population of pDCs, the majority of the functional IDO-mediated suppressor activity segregated with a novel subset of pDCs coexpressing the B-lineage marker CDl9. Thus, it was hypothesized that IDO-mediated suppression by pDCs in TDLNs creates a local microenvironment that is potently suppressive of host antitumor T cell responses (Munn, et al. , 2004, J. Clin. Invest, 114(2): 280-90).

IDO degrades the indole moiety of tryptophan, serotonin and melatonin, and initiates the production of neuroactive and immunoregulatory metabolites, collectively known as kynurenines. By locally depleting tryptophan and increasing proapoptotic kynurenines, IDO expressed by dendritic cells (DCs) can greatly affect T-cell proliferation and survival. IDO induction in DCs could be a common mechanism of deletional tolerance driven by regulatory T cells. Because such tolerogenic responses can be expected to operate in a variety of physiopathological conditions, tryptophan metabolism and kynurenine production might represent a crucial interface between the immune and nervous systems (Grohmann, et al, 2003, Trends Immunol, 24: 242-8). In states of persistent immune activation, availability of free serum Trp is diminished and, as a consequence of reduced serotonin production, serotonergic functions may also be affected (Wirleitner, et al., 2003, Curr. Med. Chem., 10: 1581-91).

TDO is mainly present in the liver and metabolizes 95% of hepatic Trp. It plays a key role in the emotional and psychiatric status. Due to the similar enzymatic function with IDO, it can also suppress inflammation and maintain immune homeostasis by Trp starvation. It was found that the TDO in HeLa cells could inhibit certain bacteria, parasites and viruses (Schmidt SK, et al., 2009, Eur J Immunol., 39: 2755-64). In animal models, TDO was also reported to suppress disease phenotypes and promote survival (Michels H, et al., 2016, Sci Rep., 6: 39199). Additionally, it was reported that, under hypoxic conditions, the ability of TDO to inhibit pathogens and T-cell proliferation could be attenuated (Elbers F, et al., 2016, Mediators Inflamm., 1638916).

It was reported that cancer tissues express IDO1 or TDO, or both (Liu X, et al., 2010, Blood. 115: 3520-30). Many studies have reported that TDO could be expressed in various cancers, such as brain cancers, lung cancers and breast cancers (Abdel-Magid AF., 2017, ACS Med Chem Lett., 8: 11-3).

Due to the similar roles of TDO and IDO in regulating the Kyn pathway, TDO was considered a potential therapeutic target to treat cancer. TDO has been shown to have immunomodulatory functions in promoting tumor immune resistance and proliferation (Pantouris G, et al., 2016, J Enzyme Inhib Med Chem., 31: 70-8). Some data have suggested that cancer cells overexpressing TDO and activating AhR can evade immune surveillance (Cheong JE, et al, 2018, Trends Pharmacol Sci., 39: 307-25).

In light of the potential role for IDO and/or TDO in immunosuppression, tumor resistance and/or rejection, chronic infections, HIV-infection, AIDS (including its manifestations such as cachexia, dementia and diarrhea), autoimmune diseases or disorders (such as rheumatoid arthritis), and immunologic tolerance and prevention of fetal rejection in utero, therapeutic agents aimed at suppression of tryptophan degradation by inhibiting IDO and/or TDO activity are desirable. Inhibitors of IDO and/or TDO can be used to activate T cells and therefore enhance T cell activation when the T cells are suppressed by pregnancy, malignancy or a virus such as HIV. Inhibition of IDO and/or TDO may also be an important treatment strategy for subjects with neurological or neuropsychiatric diseases or disorders such as depression.

The novel substituted 1,3,8-triazaspiro[4.5]decane-2,4-dione compounds disclosed herein are potent IDO and TDO inhibitors and are useful in the potential treatment or prevention of IDO- and/or TDO-associated diseases. These compounds also have unexpected properties over known IDO and/or TDO inhibitors.

## SUMMARY OF THE INVENTION

The invention is defined by the claims and relates to a novel substituted 1,3,8-triazaspiro[4.5]decane-2,4-dione compound, as defined in claim 1 which is a potent inhibitor of the IDO and TDO enzymes. The invention also relates to the compound for use in therapy, pharmaceutical compositions comprising the compound, and combinations of the compound and one or more other active agents.

## DETAILED DESCRIPTION OF THE INVENTION

The compound of the invention is:

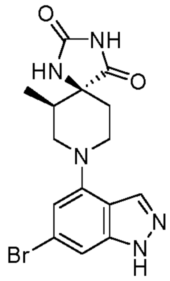

,

or a pharmaceutically acceptable salt thereof.

In one embodiment of the compound of formula (I), the compound is:

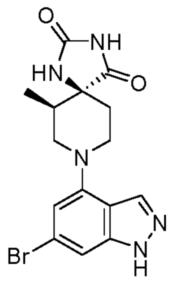

.

Also disclosed herein is a pharmaceutical composition comprising a compound disclosed herein and at least one pharmaceutically acceptable carrier.

Also disclosed herein is the compound of the invention for use in a method of inhibiting activity of indoleamine 2,3-dioxygenase (IDO) and/or tryptophan 2,3-dioxygenase (TDO) comprising contacting IDO and/or TDO receptors with a compound disclosed herein, or a pharmaceutically acceptable salt, solvate or hydrate thereof.

Also disclosed herein is the compound of the invention for use in a method of inhibiting immunosuppression in a subject comprising administering to said subject an effective amount of a compound disclosed herein, or a pharmaceutically acceptable salt, solvate or hydrate thereof.

Also disclosed herein is the compound of the invention for use in a method of treating cancer, viral infection, depression, a neurodegenerative disorder, trauma, age-related cataracts, organ transplant rejection, or an autoimmune disease in a subject comprising administering to said subject an effective amount of a compound disclosed herein, or a pharmaceutically acceptable salt, solvate or hydrate thereof. In one embodiment, the method is for treating cancer in a subject. In one embodiment, the method is for treatment viral infection in a subject. In one embodiment, the method is for treating depression in a subject. In one embodiment, the method is for treating a neurodegenerative disorder in a subject. In one embodiment, the method is for treating trauma in a subject. In one embodiment, the method is for treating age-related cataracts in a subject. In one embodiment, the method is for treating organ transplant rejection in a subject. In one embodiment, the method is for treating an autoimmune disease in a subject

Also disclosed herein is the compound of the invention for use in a method of treating melanoma in a subject comprising administering to said subject an effective amount of a compound disclosed herein, or a pharmaceutically acceptable salt, solvate or hydrate thereof.

Also disclosed herein is the compound of the invention for use in a method of treating head and neck cancer in a subject comprising administering to said subject an effective amount of a compound disclosed herein, or a pharmaceutically acceptable salt, solvate or hydrate thereof.

Compounds described herein, for example, Compound 1 or the claimed compound, Compound 1-1 having the following structures, are potent inhibitors of both IDO and TDO enzymes.

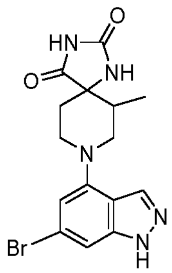 Compound 1; 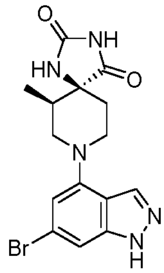 Compound 1-1.

Additionally, these compounds have unexpected properties over known compounds with regard to specificity for IDO and TDO enzymes over off-target kinases. In one embodiment, compound 1-1 exhibits potent inhibition of IDO and TDO receptors while showing less activities against one more more off-target kinases selected from CDK2, CDK5, CDK9, CLK2, FLT3, LRRK2, MELK, RPS6KB1, SGK2 and TYK2, as compared to known compounds. In one embodiment, the comparative compound is CC-1 which is disclosed as compound 137 in PCT publication WO2016071293.

Instant compounds can also have improved aqueous solubility over known compounds. In one embodiment, instant compound 1-1 has higher aqueous solubility than known compound CC-1.

Further disclosed herein is an instant compound, or a pharmaceutically acceptable salt thereof, for use in therapy. In particular embodiments, the therapy is for the treatment of a disease or disorder associated with an IDO- and/or TDO-associated disease or disorder. In particular embodiments the therapy is for the treatment of an IDO-associated disease or disorder. In particular embodiments, the therapy is for the treatment of an IDO-associated disease or disorder specifically set forth in the instant application.

"Alkyl" refers to both branched- and straight-chain saturated aliphatic hydrocarbon groups of 1 to 18 carbon atoms, or more specifically, 1 to 12 carbon atoms. Examples of such groups include, but are not limited to, methyl (Me), ethyl (Et), n-propyl (Pr), n-butyl (Bu), n-pentyl, n-hexyl, and the isomers thereof such as isopropyl (i-Pr), isobutyl (i-Bu), sec-butyl (s-Bu), tert-butyl (t-Bu), isopentyl, and isohexyl. Alkyl groups may be optionally substituted with one or more substituents selected from halogen and -OH. "$C_{1-6}$alkyl" refers to an alkyl group as defined herein having 1 to 6 carbon atoms.

"Halo" or "halogen" refers to fluoro, chloro, bromo or iodo, unless otherwise noted. In one embodiment, halogen is selected from fluoro, chloro and bromo. In one embodiment, halogen is selected from chloro and bromo. In one embodiment, halogen is bromo. In one embodiment, halogen is chloro.

"Optionally substituted" refers to "unsubstituted or substituted," and therefore, the generic structural formulas described herein encompass compounds containing the specified optional substituent(s) as well as compounds that do not contain the optional substituent(s). Each substituent is independently defined each time it occurs within the generic structural formula definitions.

Polymorphism

A compound disclosed herein, including a salt, solvate or hydrate thereof, may exist in crystalline form, non-crystalline form, or a mixture thereof. A compound or a salt or solvate thereof may also exhibit polymorphism, i.e. the capacity of occurring in different crystalline forms. These different crystalline forms are typically known as "polymorphs". Polymorphs have the same chemical composition but differ in packing, geometrical arrangement, and other descriptive properties of a crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, all of which may be used for identification. One of ordinary skill in the art will appreciate that different polymorphs may be produced, for example, by changing or adjusting the conditions used in crystallizing/recrystallizing a compound disclosed herein.

Optical Isomers - Diastereomers - Geometric Isomers - Tautomers

Included herein are various isomers of the compounds disclosed herein. The term "isomers" refers to compounds that have the same composition and molecular weight but differ in physical and/or chemical properties. The structural difference may be in constitution (geometric isomers) or in the ability to rotate the plane of polarized light (stereoisomers).

With regard to stereoisomers, a compound disclosed herein may have one or more asymmetric carbon atom and may occur as mixtures (such as a racemic mixture) or as individual enantiomers or diastereomers. All such isomeric forms are included herein, including mixtures thereof. If a compound disclosed herein contains a double bond, the substituent may be in the E or Z configuration. If a compound disclosed herein contains a disubstituted cycloalkyl, the cycloalkyl substituent may have a cis- or trans- configuration. All tautomeric forms are also intended to be included.

Any asymmetric atom (e.g., carbon) of a compound disclosed herein, can be present in racemic mixture or enantiomerically enriched, for example the (R)-, (S)- or (R,S)-configuration. In certain embodiments, each asymmetric atom has at least 50 % enantiomeric excess, at least 60 % enantiomeric excess, at least 70 % enantiomeric excess, at least 80 % enantiomeric excess, at least 90 % enantiomeric excess, at least 95 % enantiomeric excess, or at least 99 % enantiomeric excess in the (R)- or (S)- configuration. Substituents at atoms with unsaturated double bonds may, if possible, be present in cis- (Z)- or trans- (E)- form.

A compound disclosed herein, can be in the form of one of the possible isomers, rotamers, atropisomers, tautomers or mixtures thereof, for example, as substantially pure geometric (cis or trans) isomers, diastereomers, optical isomers (antipodes), racemates or mixtures thereof.

Any resulting mixtures of isomers can be separated on the basis of the physicochemical differences of the constituents, into the pure or substantially pure geometric or optical isomers, diastereomers, racemates, for example, by chromatography and/or fractional crystallization.

Any resulting racemates of the final compounds of the examples or intermediates can be resolved into the optical antipodes by known methods, e.g., by separation of the diastereomeric salts thereof, obtained with an optically active acid or base, and liberating the optically active acidic or basic compound. In particular, a basic moiety may thus be employed to resolve the compounds of the present invention into their optical antipodes, e.g., by fractional crystallization of a salt formed with an optically active acid, e.g., tartaric acid, dibenzoyl tartaric acid, diacetyl tartaric acid, di-O,O'-*p*-toluoyl tartaric acid, mandelic acid, malic acid or camphor-10-sulfonic acid. Racemic compounds can also be resolved by chiral chromatography, e.g., high pressure liquid chromatography (HPLC) using a chiral adsorbent.

Some of the compounds described herein may exist with different points of attachment of hydrogen, referred to as tautomers. For example, compounds including carbonyl $-CH_2C(O)-$ groups (keto forms) may undergo tautomerism to form hydroxyl $-CH=C(OH)-$ groups (enol forms). Both keto and enol forms, individually as well as mixtures thereof, are included within the scope of the present invention.

Isotopic Variations

Compounds disclosed herein, include unlabeled forms, as well as isotopically labeled forms. Isotopically labeled compounds have structures depicted by the formulas given herein except that one or more atoms are replaced by an atom having a selected atomic mass or mass number. Examples of isotopes that can be incorporated into compounds disclosed herein include isotopes of hydrogen, carbon, nitrogen, oxygen, phosphorous, sulfur, fluorine, iodine and chlorine, such as $^{2}H$ (i.e., Deuterium or "D"), $^{3}H$, $^{11}C$, $^{13}C$, $^{14}C$, $^{13}N$, $^{15}N$, $^{15}O$, $^{17}O$, $^{18}O$, $^{32}P$, $^{35}S$, $^{18}F$, $^{123}I$, $^{125}I$ and $^{36}Cl$. The invention includes various isotopically labeled compounds as defined herein, for example those into which radioactive isotopes, such as $^{3}H$ and $^{14}C$, or those into which non-radioactive isotopes, such as $^{2}H$ and $^{13}C$ are present. Such isotopically labeled compounds are useful in metabolic studies (with $^{14}C$), reaction kinetic studies (with, for example $^{2}H$ or $^{3}H$), detection or imaging techniques, such as positron emission tomography (PET) or single-photon emission computed tomography (SPECT) including drug or substrate tissue distribution assays, or in radioactive treatment of subjects. In particular, substitution with positron emitting isotopes, such as $^{11}C$, $^{18}F$, $^{15}O$ and $^{13}N$, may be particularly desirable for PET or SPECT studies.

Isotopically-labeled compounds disclosed herein, can generally be prepared by conventional techniques known to those skilled in the art. Furthermore, substitution with heavier isotopes, particularly deuterium (i.e., $^{2}H$ or D) may afford certain therapeutic advantages resulting from greater metabolic stability, for example increased in vivo half-life or reduced dosage requirements or an improvement in therapeutic index.

Pharmaceutically Acceptable Salts

The term "pharmaceutically acceptable salt" refers to a salt prepared from a pharmaceutically acceptable non-toxic base or acid, including inorganic or organic base and inorganic or organic acid. Salts derived from inorganic bases include aluminum, ammonium, calcium, copper, ferric, ferrous, lithium, magnesium, manganic salts, manganous, potassium, sodium, zinc, and the like. Particular embodiments include ammonium, calcium, magnesium, potassium, and sodium salts. Salts in the solid form may exist in more than one crystal structure, and may also be in the form of hydrates. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylene-diamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

When a compound disclosed herein is basic, a salt may be prepared from a pharmaceutically acceptable non-toxic acid, including an inorganic and organic acid. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, trifluoroacetic acid (TFA) and the like. Particular embodiments include the citric, hydrobromic, hydrochloric, maleic, phosphoric, sulfuric, fumaric, tartaric and trifluoroacetic acids.

Methods of Use (to be interpreted as compounds for use in such methods)

Compounds disclosed herein can inhibit activity of the enzyme indoleamine-2,3-dioxygenase (IDO) and/or the enzyme tryptophan-2,3- dioxygenase (IDO). For example, the compound of the invention can be for use in inhibiting activity of IDO and/or TDO in cell or in an individual in need of modulation of the enzymes by administering an effective amount of a compound. Further disclosed herein is the compound of the invention for use in methods of inhibiting the degradation of tryptophan in a system containing cells expressing IDO or TDO such as a tissue, living organism, or cell culture. In some embodiments, the present invention provides the compound of the invention for use in methods of altering (e.g., increasing) extracellular tryptophan levels in a mammal by administering an effective amount of a compound or composition provided herein. Methods of measuring tryptophan levels and tryptophan degradation are routine in the art.

Also disclosed herein is the compound of the invention for use in methods of inhibiting immunosuppression such as IDO-associated immunosuppression in a subject by administering to the subject an effective amount of a compound or composition recited herein. IDO-mediated immunosuppression has been associated with, for example, cancers, tumor growth, metastasis, viral infection, viral replication, etc.

Also disclosed herein is the compound of the invention for use in methods of treating diseases or disorders associated with activity or expression, including abnormal activity and/or overexpression, of IDO and/or TDO in an individual (e.g., subject) by administering to the individual in need of such treatment an effective amount or dose of a compound disclosed herein or a pharmaceutical composition thereof. Example diseases or disorders can include any disease, disorder or condition that may be directly or indirectly linked to expression or activity of the IDO enzyme, such as over expression or abnormal activity. An IDO-associated disease or disorder can also include any disease, disorder or condition that may be prevented, ameliorated, or cured by modulating enzyme activity. Examples of IDO-associated diseases or disorders include cancer, viral infection such as HIV and HCV, depression, neurodegenerative disorders such as Alzheimer’s disease and Huntington’s disease, trauma, age-related cataracts, organ transplantation (e.g., organ transplant rejection), and autoimmune diseases including asthma, rheumatoid arthritis, multiple sclerosis, allergic inflammation, inflammatory bowel disease, psoriasis and systemic lupus erythematosusor. Example cancers potentially treatable by the methods herein include cancer of the colon, pancreas, breast, prostate, lung, brain, ovary, cervix, testes, renal, head and neck, lymphoma, leukemia, melanoma, and the like. The compounds of the invention may also be useful in the treatment of obesity and ischemia. As used herein, the term "cell" is meant to refer to a cell that is in vitro, ex vivo or in vivo. In some embodiments, an ex vivo cell can be part of a tissue sample excised from an organism such as a mammal. In some embodiments, an in vitro cell can be a cell in a cell culture. In some embodiments, an in vivo cell is a cell living in an organism such as a mammal.

In one embodiment, an IDO-associated disease or disorder is selected from cancer, viral infection, HCV infection, depression, neurodegenerative disorder, trauma, age-related cataracts, organ transplantation, and autoimmune disease.

In one embodiment, an IDO-associated disease or disorder is a cancer selected from colon cancer, pancreas cancer, breast cancer, prostate cancer, lung cancer, brain cancer, ovary cancer, cervix cancer, testes cancer, renal cancer, head and neck cancer, lymphoma, leukemia, and melanoma.

In one embodiment, a TDO-associated disease and condition is selected from cancer, depression, neurodegenerative disorder, schizophrenia and anxiety. In one embodiment, a TDO-associated disease and condition is a cancer selected from brain cancer, lung cancer and breast cancer.

As used herein, the term "contacting" refers to the bringing together of indicated moieties in an in vitro system or an in vivo system. For example, "contacting" the IDO enzyme with a compound disclosed herein includes the administration of a compound of the present invention to an individual or subject, such as a human, as well as, for example, introducing a compound of the invention into a sample containing a cellular or purified preparation containing the IDO enzyme.

A subject administered with a compound disclosed herein, or a pharmaceutically acceptable salt, solvate or hydrate thereof, is generally a mammal, such as a human being, male or female. A subject also refers to cows, sheep, goats, horses, dogs, cats, rabbits, rats, mice, fish, and birds. In one embodiment, the subject is a human.

As used herein, the terms "treatment" and "treating" refer to all processes wherein there may be a slowing, interrupting, arresting, controlling, or stopping of the progression of a disease or disorder that may be associated with IDO enzyme activity. The terms do not necessarily indicate a total elimination of all disease or disorder symptoms. The terms also include the potential prophylactic therapy of the mentioned conditions, particularly in a subject that is predisposed to such disease or disorder.

The terms "administration of" and or "administering a" compound should be understood to include providing a compound described herein, or a pharmaceutically acceptable salt, solvate or hydrate thereof, and compositions of the foregoing to a subject.

The amount of a compound administered to a subject is an amount sufficient to inhibit IDO enzyme activity in the subject. In an embodiment, the amount of a compound can be an "effective amount", wherein the subject compound is administered in an amount that will elicit a biological or medical response of a tissue, system, animal or human that is being sought by a researcher, veterinarian, medical doctor or other clinician. An effective amount does not necessarily include considerations of toxicity and safety related to the administration of a compound. It is recognized that one skilled in the art may affect physiological disorders associated with an IDO enzyme activity by treating a subject presently afflicted with the disorders, or by prophylactically treating a subject likely to be afflicted with the disorders, with an effective amount of a compound disclosed herein, or a pharmaceutically acceptable salt, solvate or hydrate thereof.

An effective amount of a compound will vary with the particular compound chosen (e.g. considering the potency, efficacy, and/or half-life of the compound); the route of administration chosen; the condition being treated; the severity of the condition being treated; the age, size, weight, and physical condition of the subject being treated; the medical history of the subject being treated; the duration of the treatment; the nature of a concurrent therapy; the desired therapeutic effect; and like factors and can be routinely determined by the skilled artisan.

The compounds disclosed herein may be administered by any suitable route including oral and parenteral administration. Parenteral administration is typically by injection or infusion and includes intravenous, intramuscular, and subcutaneous injection or infusion.

The compounds disclosed herein may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound disclosed herein depend on the pharmacokinetic properties of that compound, such as absorption, distribution and half-life which can be determined by a skilled artisan. In addition, suitable dosing regimens, including the duration such regimens are administered, for a compound disclosed herein depend on the disease or condition being treated, the severity of the disease or condition, the age and physical condition of the subject being treated, the medical history of the subject being treated, the nature of concurrent therapy, the desired therapeutic effect, and like factors within the knowledge and expertise of the skilled artisan. It will be further understood by such skilled artisans that suitable dosing regimens may require adjustment given an individual subject's response to the dosing regimen or over time as the individual subject needs change. Typical daily dosages may vary depending upon the particular route of administration chosen. Typical daily dosages for oral administration, to a human weighing approximately 70 kg would range from about 0.1 mg to about 2 grams, or more specifically, 0.1 mg to 500 mg, or even more specifically, 0.2 mg to 100 mg, of a compound disclosed herein.

One embodiment of the present invention provides for the compound of the invention for use in a method of treating a disease or disorder associated with IDO enzyme activity comprising administration of an effective amount of a compound disclosed herein to a subject in need of treatment thereof. In one embodiment, the disease or disorder associated with an IDO enzyme is a cell proliferation disorder.

In one embodiment, disclosed herein is the compound of the invention for use in therapy. The compound may be useful in a method of inhibiting IDO enzyme activity in a subject, such as a mammal in need of such inhibition, comprising administering an effective amount of the compound to the subject.

In one embodiment, disclosed herein is a pharmaceutical composition comprising a compound disclosed herein, or a pharmaceutically acceptable salt, solvate or hydrate thereof, for use in potential treatment of a disorder or disease associated to IDO and/or enzyme activity.

Compositions

The term "composition" as used herein is intended to encompass a dosage form comprising a specified compound in a specified amount, as well as any dosage form which results, directly or indirectly, from a combination of a specified compound in a specified amount. Such term is intended to encompass a dosage form comprising a compound disclosed herein, or a pharmaceutically acceptable salt, solvate or hydrate thereof, and one or more pharmaceutically acceptable carriers or excipients. Accordingly, the compositions of the present invention encompass any composition made by admixing a compound of the present invention and one or more pharmaceutically acceptable carrier or excipients. By "pharmaceutically acceptable" it is meant the carriers or excipients are compatible with the compound disclosed herein and with other ingredients of the composition.

**In** one embodiment, disclosed herein is a composition comprising a compound disclosed herein, or a pharmaceutically acceptable salt, solvate or hydrate thereof, and one or more pharmaceutically acceptable carriers or excipients. The composition may be prepared and packaged in bulk form wherein an effective amount of a compound of the invention can be extracted and then given to a subject, such as with powders or syrups. Alternatively, the composition may be

prepared and packaged in unit dosage form wherein each physically discrete unit contains an effective amount of a compound disclosed herein. When prepared in unit dosage form, the composition of the invention typically contains from about 0.1 mg to 2 grams, or more specifically, 0.1 mg to 500 mg, or even more specifically, 0.2 mg to 100 mg, of a compound disclosed herein, or a pharmaceutically acceptable salt, solvate or hydrate thereof.

A compound disclosed herein and a pharmaceutically acceptable carrier or excipient(s) will typically be formulated into a dosage form adapted for administration to a subject by a desired route of administration. For example, dosage forms include those adapted for (1) oral administration, such as tablets, capsules, caplets, pills, troches, powders, syrups, elixirs, suspensions, solutions, emulsions, sachets, and cachets; and (2) parenteral administration, such as sterile solutions, suspensions, and powders for reconstitution. Suitable pharmaceutically acceptable carriers or excipients will vary depending upon the particular dosage form chosen. **In** addition, suitable pharmaceutically acceptable carriers or excipients may be chosen for a particular function that they may serve in the composition. For example, certain pharmaceutically acceptable carriers or excipients may be chosen for their ability to facilitate the production of uniform dosage forms. Certain pharmaceutically acceptable carriers or excipients may be chosen for their ability to facilitate the production of stable dosage forms. Certain pharmaceutically acceptable carriers or excipients may be chosen for their ability to facilitate the carrying or transporting of a compound disclosed herein, once administered to the subject, from one organ or portion of the body to another organ or another portion of the body. Certain pharmaceutically acceptable carriers or excipients may be chosen for their ability to enhance subject compliance.

Suitable pharmaceutically acceptable excipients include the following types of excipients: diluents, lubricants, binders, disintegrants, fillers, glidants, granulating agents, coating agents, wetting agents, solvents, co-solvents, suspending agents, emulsifiers, sweeteners, flavoring agents, flavor masking agents, coloring agents, anti-caking agents, hemectants, chelating agents, plasticizers, viscosity increasing agents, antioxidants, preservatives, stabilizers, surfactants, and buffering agents.

A skilled artisan possesses the knowledge and skill in the art to select suitable pharmaceutically acceptable carriers and excipients in appropriate amounts for the use in the invention. In addition, there are a number of resources available to the skilled artisan, which describe pharmaceutically acceptable carriers and excipients and may be useful in selecting suitable pharmaceutically acceptable carriers and excipients. Examples include Remington's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The compositions of the invention are prepared using techniques and methods known to those skilled in the art. Some methods commonly used in the art are described in Remington's Pharmaceutical Sciences (Mack Publishing Company).

The composition may be a solid oral dosage form such as a tablet or capsule comprising an effective amount of a compound of the invention and a diluent or filler. Suitable diluents and fillers include lactose, sucrose, dextrose, mannitol, sorbitol, starch (e.g. corn starch, potato starch, and pre-gelatinized starch), cellulose and its derivatives, (e.g. microcrystalline cellulose), calcium sulfate, and dibasic calcium phosphate. The oral solid dosage form may further comprise a binder. Suitable binders include starch (e.g. corn starch, potato starch, and pre-gelatinized starch) gelatin, acacia, sodium alginate, alginic acid, tragacanth, guar gum, povidone, and cellulose and its derivatives (e.g. microcrystalline cellulose). The oral solid dosage form may further comprise a disintegrant. Suitable disintegrants include crospovidone, sodium starch glycolate, croscarmelose, alginic acid, and sodium carboxymethyl cellulose. The oral solid dosage form may further comprise a lubricant. Suitable lubricants include stearic acid, magnesium stearate, calcium stearate, and talc.

Where appropriate, dosage unit formulations for oral administration can be microencapsulated. The composition can also be prepared to prolong or sustain the release as, for example, by coating or embedding particulate material in polymers, wax, or the like.

The compounds disclosed herein may also be coupled with soluble polymers as targetable drug carriers. Such polymers can include polyvinylpyrrolidone, pyrancopolymer, polyhydroxypropylmethacrylamidephenol, polyhydroxyethylaspartamidephenol, or polyethyleneoxidepolylysine substituted with palmitoyl residues. Furthermore, the compounds of the invention may be coupled to a class of biodegradable polymers useful in achieving controlled release of a drug, for example polylactic acid, polepsilon caprolactone, polyhydroxy butyric acid, polyorthoesters, polyacetals, polydihydropyrans, polycyanacrylates and crosslinked or amphipathic block copolymers of hydrogels.

The composition may also be a liquid oral dosage form. Oral liquids such as solution, syrups and elixirs can be prepared in dosage unit form so that a given quantity contains a predetermined amount of a compound disclosed herein. Syrups can be prepared by dissolving the compound of the invention in a suitably flavored aqueous solution; while elixirs are prepared through the use of a non-toxic alcoholic vehicle. Suspensions can be formulated by dispersing a compound disclosed herein in a non-toxic vehicle. Solubilizers and emulsifiers such as ethoxylated isostearyl alcohols and polyoxy ethylene sorbitol ethers, preservatives, flavor additives such as peppermint oil or other natural sweeteners or saccharin or other artificial sweeteners and the like can also be added.

The compositions may also be for parenteral administration. Compositions adapted for parenteral administration include aqueous and non-aqueous sterile injection solutions which may contain anti-oxidants, buffers, bacteriostats and

solutes which render the formulation isotonic with the blood of the intended recipient; and aqueous and non-aqueous sterile suspensions which may include suspending agents and thickening agents. The compositions may be presented in unit-dose or multi-dose containers, for example sealed ampoules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Combinations

A compound disclosed herein may be used in combination with one or more other active agents, including but not limited to, other anti-cancer agents, that are used in the prevention, treatment, control, amelioration, or reduction of risk of a particular disease or condition (e.g., cell proliferation disorders). In one embodiment, a compound disclosed herein is combined with one or more other anti-cancer agents for use in the prevention, treatment, control amelioration, or reduction of risk of a particular disease or condition for which the compounds disclosed herein are useful. Such other active agents may be administered, by a route and in an amount commonly used therefor, contemporaneously or sequentially with a compound of the present invention.

When a compound disclosed herein is used contemporaneously with one or more other active agents, a composition containing such other active agents in addition to the compound disclosed herein is contemplated. Accordingly, the compositions of the present invention include those that also contain one or more other active ingredients, in addition to a compound disclosed herein. A compound disclosed herein may be administered either simultaneously with, or before or after, one or more other therapeutic agent(s). A compound disclosed herein may be administered separately, by the same or different route of administration, or together in the same pharmaceutical composition as the other agent(s).

Products provided as a combined preparation include a composition comprising a compound disclosed herein and one or more other active agent(s) together in the same pharmaceutical composition, or a compound disclosed herein, and one or more other therapeutic agent(s) in separate form, e.g. in the form of a kit.

The weight ratio of a compound disclosed herein to a second active agent may be varied and will depend upon the effective dose of each agent. Generally, an effective dose of each will be used. Thus, for example, when a compound disclosed herein is combined with another agent, the weight ratio of the compound disclosed herein to the other agent will generally range from about 1000:1 to about 1:1000, such as about 200:1 to about 1:200. Combinations of a compound disclosed herein and other active agents will generally also be within the aforementioned range, but in each case, an effective dose of each active agent should be used. In such combinations, the compound disclosed herein and other active agents may be administered separately or in conjunction. In addition, the administration of one element may be prior to, concurrent to, or subsequent to the administration of other agent(s).

In one embodiment, the invention provides a composition comprising a compound disclosed herein, and at least one other therapeutic agent as a combined preparation for simultaneous, separate or sequential use in therapy. In one embodiment, the therapy is the treatment of a disease or disorder associated with IDO enzyme activity.

A further possibility is a kit comprising two or more separate pharmaceutical compositions, at least one of which contains a compound disclosed herein. In one embodiment, the kit comprises means for separately retaining said compositions, such as a container, divided bottle, or divided foil packet. An example of such a kit is a blister pack, as typically used for the packaging of tablets, capsules and the like.

A kit disclosed herein may be used for administering different dosage forms, for example, oral and parenteral, for administering the separate compositions at different dosage intervals, or for titrating the separate compositions against one another. To assist with compliance, a kit typically comprises directions for administration.

Disclosed herein is a use of a compound disclosed herein, for treating a disease or disorder associated with IDO enzyme activity, wherein the medicament is prepared for administration with another active agent. Also possible is the use of another active agent for treating a disease or disorder associated with an IDO enzyme, wherein the medicament is administered with a compound disclosed herein.

The disclosure also provides the use of a compound disclosed herein for treating a disease or disorder associated with IDO enzyme activity, wherein the subject has previously (e.g. within 24 hours) been treated with another active agent. The disclosure also provides the use of another therapeutic agent for treating a disease or disorder associated with IDO enzyme activity, wherein the subject has previously (e.g. within 24 hours) been treated with a compound disclosed herein. The second agent may be applied a week, several weeks, a month, or several months after the administration of a compound disclosed herein.

In one embodiment, the other active agent is selected from the group consisting of vascular endothelial growth factor (VEGF) receptor inhibitors, topoisomerase II inhibitors, smoothen inhibitors, alkylating agents, anti-tumor antibiotics, anti-metabolites, retinoids, immunomodulatory agents including but not limited to anti-cancer vaccines, CTLA-4, LAG-3 and PD-1 antagonists.

Examples of vascular endothelial growth factor (VEGF) receptor inhibitors include, but are not limited to, bevacizumab (sold under the trademark AVASTIN by Genentech/Roche), axitinib, (N-methyl-2-[[3-[([pound])-2-pyridin-2-ylethenyl]-1 H-indazol-6-yl]sulfanyl]benzamide, also known as AG013736, and described in PCT Publication No. WO 01 /002369), Brivanib Alaninate ((S)-((R)-1-(4-(4-Fluoro-2-methyl-1H-indol-5-yloxy)-5-methylpyrrolo[2,1-f][1,2,4]triazin-6-yloxy)propan-2-yl)2-aminopropanoate, also known as BMS-582664), motesanib (N-(2,3-dihydro-3,3-dimethyl-1 H-indoi-6-yl)-2-[(4-pyridinyimethyj)amino]-3-pyfidinecarboxamide. and described in PCT Publication No. WO 02/068470), pasireotide (also known as SO 230, and described in PCT Publication No. WO 02/010192), and sorafenib (sold under the tradename NEXAVAR).

Examples of topoisomerase II inhibitors, include but are not limited to, etoposide (also known as VP-16 and Etoposide phosphate, sold under the tradenames TOPOSAR, VEPESID and ETOPOPHOS), and teniposide (also known as VM-26, sold under the tradename VUMON).

Examples of alkylating agents, include but are not limited to, 5-azacytidine (sold under the trade name VIDAZA), decitabine (sold under the trade name of DECOGEN), temozolomide (sold under the trade names TEMODAR and TEMODAL by Schering-Plough/Merck), dactinomycin (also known as actinomycin-D and sold under the tradename COSMEGEN), melphalan (also known as L-PAM, L-sarcolysin, and phenylalanine mustard, sold under the tradename ALKERAN), altretamine (also known as hexamethylmelamine (HMM), sold under the tradename HEXALEN), carmustine (sold under the tradename BCNU), bendamustine (sold under the tradename TREANDA), busulfan (sold under the tradenames BUSULFEX and MYLERAN), carboplatin (sold under the tradename PARAPLATIN), lomustine (also known as CCNU, sold under the tradename CeeNU), cisplatin (also known as CDDP, sold under the tradenames PLATINOL and PLATINOL-AQ), chlorambucil (sold under the tradename LEUKERAN), cyclophosphamide (sold under the tradenames CYTOXAN and NEOSAR), dacarbazine (also known as DTIC, DIC and imidazole carboxamide, sold under the tradename DTIC-DOME), altretamine (also known as hexamethylmelamine (HMM) sold under the tradename HEXALEN), ifosfamide (sold under the tradename IFEX), procarbazine (sold under the tradename MATULANE), mechlorethamine (also known as nitrogen mustard, mustine and mechloroethamine hydrochloride, sold under the tradename MUSTARGEN), streptozocin (sold under the tradename ZANOSAR), thiotepa (also known as thiophosphoamide, TESPA and TSPA, and sold under the tradename THIOPLEX).

Examples of anti-tumor antibiotics include, but are not limited to, doxorubicin (sold under the tradenames ADRIAMYCIN and RUBEX), bleomycin (sold under the tradename LENOXANE), daunorubicin (also known as dauorubicin hydrochloride, daunomycin, and rubidomycin hydrochloride, sold under the tradename CERUBIDINE), daunorubicin liposomal (daunorubicin citrate liposome, sold under the tradename DAUNOXOME), mitoxantrone (also known as DHAD, sold under the tradename NOVANTRONE), epirubicin (sold under the tradename ELLENCE), idarubicin (sold under the tradenames IDAMYCIN, IDAMYCIN PFS), and mitomycin C (sold under the tradename MUTAMYCIN).

Examples of anti-metabolites include, but are not limited to, claribine (2-chlorodeoxyadenosine, sold under the tradename LEUSTATIN), 5-fluorouracil (sold under the tradename ADRUCIL), 6-thioguanine (sold under the tradename PURINETHOL), pemetrexed (sold under the tradename ALIMTA), cytarabine (also known as arabinosylcytosine (Ara-C), sold under the tradename CYTOSAR-U), cytarabine liposomal (also known as Liposomal Ara-C, sold under the tradename DEPOCYT), decitabine (sold under the tradename DACOGEN), hydroxyurea (sold under the tradenames HYDREA, DROXIA and MYLOCEL), fludarabine (sold under the tradename FLUDARA), floxuridine (sold under the tradename FUDR), cladribine (also known as 2-chlorodeoxyadenosine (2-CdA) sold under the tradename LEUSTATIN), methotrexate (also known as amethopterin, methotrexate sodium (MTX), sold under the tradenames RHEUMATREX and TREXALL), and pentostatin (sold under the tradename NIPENT).

Examples of retinoids include, but are not limited to, alitretinoin (sold under the tradename PANRETIN), tretinoin (all-trans retinoic acid, also known as ATRA, sold under the tradename VESANOID), Isotretinoin (13-c/s-retinoic acid, sold under the tradenames ACCUTANE, AMNESTEEM, CLARAVIS, CLARUS, DECUTAN, ISOTANE, IZOTECH, ORATANE, ISOTRET, and SOTRET), and bexarotene (sold under the tradename TARGRETIN).

"PD-1 antagonist" means any chemical compound or biological molecule that blocks binding of PD-L1 expressed on a cancer cell to PD-1 expressed on an immune cell (T cell, B cell or NKT cell) and preferably also blocks binding of PD-L2 expressed on a cancer cell to the immune-cell expressed PD-1. Alternative names or synonyms for PD-1 and its ligands include: PDCD1, PD1, CD279 and SLEB2 for PD-1; PDCD1L1, PDL1, B7H1, B7-4, CD274 and B7-H for PD-L1; and PDCD1L2, PDL2, B7-DC, Btdc and CD273 for PD-L2. In any of the treatment method, medicaments and uses of the present invention in which a human individual is being treated, the PD-1 antagonist blocks binding of human PD-L1 to human PD-1, and preferably blocks binding of both human PD-L1 and PD-L2 to human PD-1. Human PD-1 amino acid sequences can be found in NCBI Locus No.: NP_005009. Human PD-L1 and PD-L2 amino acid sequences can be found in NCBI Locus No.: NP_054862 and NP_079515, respectively.

PD-1 antagonists useful in any of the treatment method, medicaments and uses include a monoclonal antibody (mAb), or antigen binding fragment thereof, which specifically binds to PD-1 or PD-L1, and preferably specifically binds to human PD-1 or human PD-L1. The mAb may be a human antibody, a humanized antibody or a chimeric antibody, and may include a human constant region. In some embodiments the human constant region is selected from the group consisting

of IgG1, IgG2, IgG3 and IgG4 constant regions, and in preferred embodiments, the human constant region is an IgG1 or IgG4 constant region. In some embodiments, the antigen binding fragment is selected from the group consisting of Fab, Fab'-SH, $F(ab')_2$, scFv and Fv fragments. Examples of PD-1 antagonists include, but are not limited to, pembrolizumab (sold under the tradename KEYTRUDA) and nivolumab (sold under the tradename OPDIVO).

Examples of mAbs that bind to human PD-1, and useful in the treatment method, medicaments and uses are described in US7488802, US7521051, US8008449, US8354509, US8168757, WO2004/004771, WO2004/072286, WO2004/056875, and US2011/0271358.

Examples of mAbs that bind to human PD-L1, and useful in the treatment method, medicaments and uses are described in WO2013/019906, W02010/077634 A1 and US8383796. Specific anti-human PD-L1 mAbs useful as the PD-1 antagonist in the treatment method, medicaments and uses include MPDL3280A, BMS-936559, MEDI4736, MSB0010718C and an antibody which comprises the heavy chain and light chain variable regions of SEQ ID NO:24 and SEQ ID NO:21, respectively of WO2013/019906.

Other PD-1 antagonists useful in any of the treatment method, medicaments and uses include an immunoadhesin that specifically binds to PD-1 or PD-L1, and preferably specifically binds to human PD-1 or human PD-L1, e.g., a fusion protein containing the extracellular or PD-1 binding portion of PD-L1 or PD-L2 fused to a constant region such as an Fc region of an immunoglobulin molecule. Examples of immunoadhesion molecules that specifically bind to PD-1 are described in WO2010/027827 and WO2011/066342. Specific fusion proteins useful as the PD-1 antagonist in the treatment method, medicaments and uses include AMP-224 (also known as B7-DCIg), which is a PD-L2-FC fusion protein and binds to human PD-1.

Examples of other cytotoxic agents include, but are not limited to, arsenic trioxide (sold under the tradename TRISENOX), asparaginase (also known as L-asparaginase, and Erwinia L-asparaginase, sold under the tradenames ELSPAR and KIDROLASE).

EXPERIMENTAL

The following synthetic schemes and examples are intended to be illustrative only. Abbreviations used are those conventional in the art or the following.

| | |
|---|---|
| ACN | acetonitrile |
| Ac | aceyl |
| ADP | adenosine di phosphate |
| atm. | atmosphere |
| ATP | adenosine tri phosphate |
| AUC | area under the curve |
| aq. | aqueous |
| Boc | tert-butyloxycarbonyl |
| $Boc_2O$ | di-tert-butyl dicarbonate |
| Calc'd | calculated |
| CDK2 | cyclin-dependent kinase 2 |
| CDK5 | cyclin-dependent kinase 5 |
| CDK5/P25 | Cell division protein kinase 5, activated by protein p25 |
| CDK9 | cyclin-dependent kinase 9 |
| CDK9 (Cyclin T1) | cyclin-dependent kinase 9, complexed with Cyclin-T1 |
| Celite | diatomaceous earth used as a filtration medium |
| °C | degree celsius |
| CLK2 | CDC like kinase 2 |
| DCM | dichloromethane |
| DIEA | N,N-diisopropylethylamine |
| DIPEA | N,N-diisopropylethylamine |
| DMF | N,N-dimethylformamide |
| DMSO | dimethylsulfoxide |
| DPTTA | di-p-toluoyl-d-tartaric acid |
| EDTA | ethylenediaminetetraacetic acid |
| ee | enantiomeric excess |
| EI | electron ionization |
| EMEM | Eagle's minimal essential medium |
| eq. or equiv. | equivalent |
| Et | ethyl |

| | |
|---|---|
| EGTA | Ethylene glycol-bis(B-aminoethylether)-N,N,N',N'-tetraacetic acid |
| $Et_2O$ | diethyl ether |
| $Et_3N$ | triethylamine |
| EtOAc | ethyl acetate |
| EtOH | ethanol |
| FLT3(D835Y)Fms | like tyrosine kinase 3, asparagiene to tyrosine mutation at position 835 |
| g | gram |
| h | hour(s) |
| HCl | hydrochloric acid |
| HEPES | 4-(2-hydroxyethyl)-1-piperazineethanesulfonic acid |
| HESI | heated-electrospray ionization |
| HPLC | high pressure liquid chromatography |
| kg | kilogram |
| L | liter |
| LC | liquid chromatography |
| LCMS or LC/MS | liquid chromatography and mass spectrometry |
| LiHMDS | lithium bis(trimethylsilyl)amide |
| LPS | lipopolysaccharide |
| LRRK2 | leucine-rich repeat kinase 2 |
| LRRK2 (G2019S) | Leucine-rich repeat kinase 2, glycine to serine mutation at position 2019 |
| M | molar |
| Me | methyl |
| MeOH | methanol |
| mg | miligram |
| $MgSO_4$ | magnesium sulfate |
| MELK | Maternal embryonic leucine zipper kinase |
| mmol | milimole |
| MS | mass spectrometry |
| MTBE | methyl tert-butyl ether |
| min | minutes |
| mL | milliliter(s) |
| m/z | mass to charge ratio |
| nm | nanometer |
| nM | nanomolar |
| N | normal |
| $Na_2SO_4$ | sodium sulfate |
| $NaHCO_3$ | sodium bicarbonate |
| NaOH | sodium Hydroxide |
| NBS | non-binding surface |
| $NH_4Cl$ | ammonium chloride |
| NMP | N-methyl-2-pyrrolidone |
| Pd | palladium |
| $Pd(AOc)_2$ | palladium diacetate |
| Pd-C or Pd/C | palladium on carbon |
| PE | petroleum ether |
| PKC | protein kinase C |
| RPMI | Roswell Park Memorial Institute |
| RPS6KB1 | Ribosomal protein S6 kinase beta-1 |
| RT or rt | room temperature |
| RuPhos G1 | Chloro-(2-Dicyclohexylphosphino-2',6'-diisopropoxy-1,1'-biphenyl)[2-(2-aminoethyl)phenyl]palladium(II) |
| sat. | saturated |
| SGK2 | Serum/glucocorticoid regulated kinase 2 |
| SFC | supercritical fluid chromatography |
| SRM | selected-reaction monitoring |
| TEA | triethyl amine |
| TFA | trifluoroacetic acid |
| TFAA | trifluoroacetic anhydride |

| | |
|---|---|
| THF | tetrahydrofuran |
| TYK2 | tyrosine kinase 2 |
| uL | microliter(s) |

GENERAL SYNTHETIC SCHEMES

The following syntheses are representative exemplary methods by which the compounds and comparative compounds disclosed herein can be synthesized.

In the schemes described below, it is well understood that protecting groups for sensitive or reactive groups are employed where necessary in accordance with general principles of chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art.

EXAMPLES AND COMPARATIVE EXAMPLES

All syntheses may be performed using reagents and reaction conditions suitable for similar reactions known in the field. All compounds disclosed herein may be produced by employing analogous syntheses.

Some specific syntheses of compounds disclosed herein are set out in the following. Reagents were purchased from commercial sources and were used as received. $^1H$ NMR spectra were obtained on a Bruker Ultra Shield spectrometer at 600 MHz or a Varian 500 spectrometer at 499 MHz with tetramethylsilane used as an internal reference. LC/MS spectra were obtained on Agilent 6120 Quadrupole LC/MS spectrometers using electrospray ionization. HPLC analyses were performed on an Agilent 1100 Series instrument and on a Shimadzu SIL-20A instrument. Impurities are expressed as % AUC by HPLC and are non-validated.

**Synthesis of Comparative Compound CC-1**

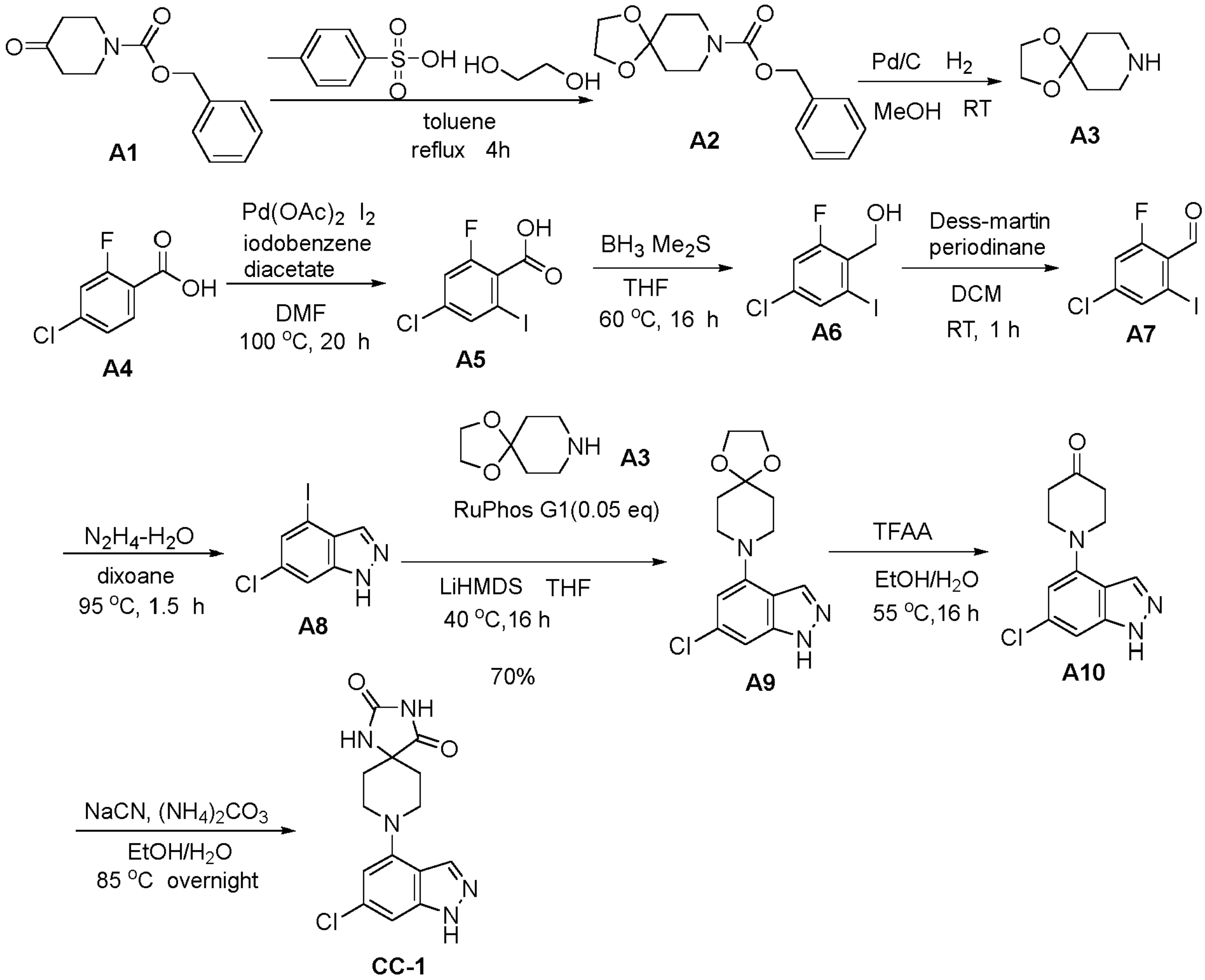


Step 1: 8-Benzyl-1,4-dioxa-8-azaspiro[4.5]decane (A2)

Into a 10000-mL 4-necked round-bottom flask, was placed a solution of benzyl 4-oxopiperidine-1-carboxylate (155 g, 664.49 mmol, 1.00 equiv) in toluene (5.500 L), 4-methylbenzene-1-sulfonic acid (183 g, 1.06 mol, 1.60 equiv), ethane-1,2-diol (330 g, 5.32 mol, 8.00 equiv). The resulting solution was heated to reflux with Dean-Stark apparatus for 4 h. The reaction mixture was cooled to RT, and the pH value of the solution was adjusted to 9 with the addition of sodium bicarbonate (sat., aqueous). The resulting solution was diluted with 5 L of $H_2O$, extracted with toluene, and the combined organic layers were concentrated under vacuum to provide compound A2, which was used without further purification.

Step 2: 1,4-Dioxa-8-azaspiro[4.5]decane (A3)

Into a 3 L 3-necked round-bottom flask, was placed benzyl 1,4-dioxa-8-azaspiro[4.5]decane-8-carboxylate (A2) (166 g, 598.60 mmol, 1.00 equiv) and palladium on carbon (50 g, 0.50 equiv). The resulting solution was stirred overnight at room temperature under an atmosphere of hydrogen (balloon, 1 atm). The solids were then filtered off, and the filtrate was concentrated to give 1,4-dioxa-8-azaspiro[4.5]decane (A3), which was used without further purification.

Step 3: 4-Chloro-2-fluoro-6-iodobenzoic acid (A5)

Into a 10 L 4-necked round-bottom flask, purged and maintained with an inert atmosphere of nitrogen, was placed a solution of 4-chloro-2-fluorobenzoic acid (A4) (450 g, 2.58 mol, 1.00 equiv) in DMF (6.5 L), iodobenzene diacetate (916.5 g, 2.83 mol, 1.10 equiv), iodine (718.5 g, 2.83 mol, 1.10 equiv), and palladium acetate (29 g, 129.17 mmol, 0.05 equiv). The resulting solution was stirred for 20 h at 100°C. After cooling to RT, the solution was diluted with EtOAc and washed three times with 1 N HCl. The aqueous layers were combined and extracted once more with EtOAc. The combined organic layers were dried over $Na_2SO_4$ and concentrated under vacuum to provide compound A5, which was used without further purification.

Step 4: (4-Chloro-2-fluoro-6-iodophenyl)methanol (A6)

Into a 20 L 4-necked round-bottom flask, purged and maintained with an inert atmosphere of nitrogen, was placed a solution of 4-chloro-2-fluoro-6-iodobenzoic acid (A5) (763 g, 2.54 mol, 1.00 equiv) in THF (13 L), followed by $BH_3$. $Me_2S$ (3.06 L, 10.00 equiv). The resulting solution was stirred for 16 h at 60°C and then cooled to RT. The reaction was then quenched by the addition of MeOH. The resulting mixture was concentrated under vacuum to provide compound A6, which was used without further purification.

Step 5: 4-Chloro-2-fluoro-6-iodobenzaldehyde (A7)

Into a 20 L 4-necked round-bottom flask, was placed a solution of (4-chloro-2-fluoro-6-iodophenyl)methanol (A6) (580 g, 2.02 mol, 1.00 equiv) in DCM (12 L) and Dess-martin periodinane (946 g, 1.10 equiv). The resulting solution was stirred for 1 h at RT, after which it was quenched by the addition of 6 L of $NaHCO_3$ (sat., aqueous). The layers were separated and the aqueous phase was extracted with DCM (2 x 6 L). The organic phases were then dried over $MgSO_4$, filtered and concentrated under reduced pressure. The crude product was purified by silica gel flash chromatography, eluting with a gradient of 0 - 25% EtOAc in petroleum ether to afford 4-chloro-2-fluoro-6-iodobenzaldehyde (A7).

Step 6: 6-Chloro-4-iodo-1H-indazole (A8)

Into a 10 L 4-necked round-bottom flask, purged and maintained with an inert atmosphere of nitrogen, was placed a solution of 4-chloro-2-fluoro-6-iodobenzaldehyde (A7) (460 g, 1.62 mol, 1.00 equiv) in dioxane (4500 mL) and hydrazine hydrate (202.5 g, 4.13 mol, 2.00 equiv). The resulting solution was stirred for 1.5 h at 95°C. After cooling to RT, the reaction mixture was poured into ice water (5 L) and extracted with EtOAc (5 L). The organic layer was washed with brine then dried over $MgSO_4$, filtered, and concentrated *in vacuo.* The residue was purified by silica gel chromatography, eluting with a gradient of 50 - 100% EtOAc in petroleum ether to afford 6-chloro-4-iodo-1H-indazole (A8).

Step 7: 8-(6-Chloro-1H-indazol-4-yl)-1,4-dioxa-8-azaspiro[4.5]decane (A9)

Into a 3 L, 3-necked round-bottom flask, was placed a solution of 6-chloro-4-iodo-1H-indazole (A8) (135 g, 484.78 mmol, 1.00 equiv) in THF (1500 mL), RuPhos G1 (17.7 g, 24.29 mmol, 0.05 equiv), and 1,4-dioxa-8-azaspiro[4.5] decane (A3) (76.4 g, 533.58 mmol, 1.10 equiv). This was followed by the addition of LiHMDS (875 mL, 1.80 equiv) dropwise with stirring at RT. The resulting solution was stirred for 1 h at RT and then further heated for 16 h at 40°C. The reaction was then cooled and diluted with EtOAc (2 L) and $NH_4Cl$ (saturated, aquoues, 2 L). The organics were washed with brine (2 L), dried, and concentrated. The residue was purified by silica gel flash chromatography, eluting with a gradient of 0 - 35% EtOAC in petroleum ether to afford 8-(6-chloro-1H-indazol-4-yl)-1,4-dioxa-8-azaspiro[4.5]decane (A9).

Step 8: 1-(6-Chloro-1H-indazol-4-yl)piperidin-4-one (A10)

Into a 3 L 3-necked round-bottom flask, purged and maintained with an inert atmosphere of nitrogen, was placed a solution of 8-(6-chloro-1H-indazol-4-yl)-1,4-dioxa-8-azaspiro[4.5]decane (A9) (100 g, 340.43 mmol, 1.00 equiv) in ethanol/$H_2O$ (1800/200 mL) and TFAA (71.5 g, 340.48 mmol, 1.00 equiv). The resulting solution was stirred for 16 h at 55°C. The reaction was then cooled and concentrated in vacuo while loading onto silica gel. The residue was purified via flash chromatography (silica gel, eluting with a gradient of 0-15% EtOAc in petroleum ether) to afford 1-(6-chloro-1H-indazol-4-yl)piperidin-4-one (A10).

Step 9: Comparative Compound CC-1

Into a 3L 3-necked round-bottom flask, purged and maintained with an inert atmosphere of nitrogen, was placed a solution of 1-(6-chloro-1H-indazol-4-yl)piperidin-4-one (A10) (68 g, 272.33 mmol, 1.00 equiv) in ethanol/$H_2O$ (1000/340 mL), $(NH_4)_2CO_3$ (496.2 g, 19.00 equiv), and sodium cyanide (53.5 g, 1.09 mol, 4.00 equiv). The resulting solution was stirred with heating overnight at 85°C. The reaction was then cooled and the reaction mixture was diluted with water (2.5 L) and EtOAc (5 L). The aqueous layer was extracted with EtOAc (5 L x 2) and the combined organics were washed with brine (5 L), dried over $MgSO_4$, and filtered. The filtrate was then concentrated *in vacuo* onto silica gel and the residue was purified via flash chromatography (silica gel), eluting with a gradient of 0 - 100% 3:1 EtOAc:EtOH in hexane to afford compound CC-1.

LCMS: 320 $[M+H]^+$ $^1H$ NMR: (400 MHz, DMSO-d6, *ppm):* $\delta$ 1.684-1.728 (2H, m), 2.020-2.103 (2H, m), 3.174-3.322 (2H, m), 3.690-3.733 (2H, m), 6.408-6.413 (1H, d), 7.063 (1H,s), 8.144 (1H,s), 8.573(1H, s), 10.724 (1H,s), 13.068 (1H, s).

**Synthesis of 8-(6-Bromo-1H-indazol-4-yl)-6-methyl-1,3,8-triazaspiro[4.5]decane-2,4-dione (Compound 1) and Compound 1-1**

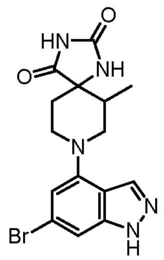

(Compound 1)

Step 1: Tert-butyl 6-methyl-2,4-dioxo-1,3,8-triazaspiro[4.5]decane-8-carboxylate

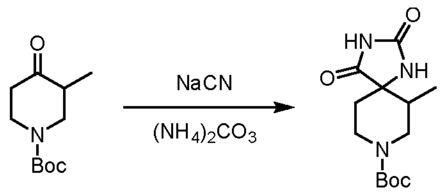


A mixture of tert-butyl 3-methyl-4-oxopiperidine-1-carboxylate (5 g, 23.44 mmol), ammonium carbonate (11.26 g, 117 mmol) and sodium cyanide (2.298 g, 46.9 mmol) in ethanol (50 ml) and water (50 ml) was sealed in a pressure tube and was stirred at 60 °C for 12 h. After cooling to RT, the mixture was concentrated and a precipitate was formed. The precipitate was collected by filtration, washed with water (200 mL) to afford the title compound as a solid, which was used directly in the next step. LCMS: 284 $[M+H]^+$

Step 2. 6-Methyl-1,3,8-triazaspiro[4.5]decane-2,4-dione hydrochloride

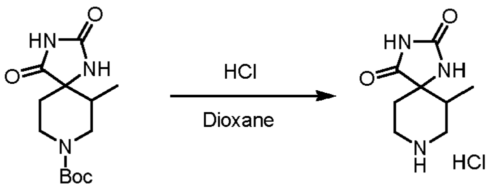


To tert-butyl 6-methyl-2,4-dioxo-1,3,8-triazaspiro[4.5]decane-8-carboxylate (1000 mg, 3.53 mmol) was added hydrochloric acid (8824 μl, 35.3 mmol) in dioxane. The reaction mixture was stirred for 2 h and LCMS indicated that the reaction was complete. Ethyl ether (50 ml) was added and a precipitate was formed which was collected by filtration. The solid was washed with ethyl ether (3 × 20 ml) and the solid was dried under vacuum to give the title compound as a solid. LCMS: 184 $[M+H]^+$

Step 3. 4-Bromo-2-fluoro-6-(6-methyl-2,4-dioxo-1,3,8-triazaspiro[4.5]decan-8-yl) benzaldehyde

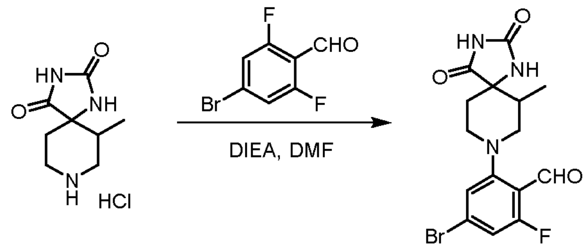


6-Methyl-1,3,8-triazaspiro[4.5]decane-2,4-dione hydrochloride (360 mg, 1.639 mmol) and 4-bromo-2,6-difluorobenzaldehyde (362 mg, 1.639 mmol) were combined in a 2-5 ml microwave vial under argon, and DMF (3 ml) and DIEA (0.859 ml, 4.92 mmol) were added. The reaction was sealed and heated to 100 °C overnight. After the reaction was cooled, it was partitioned between water and EtOAc. The mixture was extracted with EtOAc and dried with brine and sodium sulfate. After the removal of solvent, the residue was dry-loaded onto silica gel and eluted through a 12g column using 0-70% EtOAc in hexane to give the title compound as a solid. LCMS: 384 $[M+H]^+$

Step 4. 8-(6-Bromo-1H-indazol-4-yl)-6-methyl-1,3,8-triazaspiro[4.5]decane-2,4-dione (Compound 1)

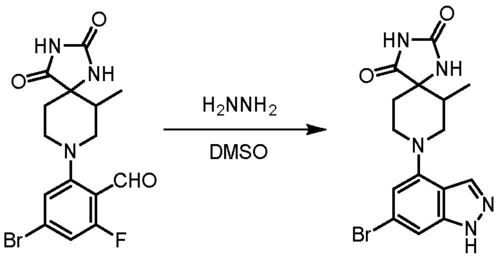

(Compound 1)

4-Bromo-2-fluoro-6-(6-methyl-2,4-dioxo-1,3,8-triazaspiro[4.5]decan-8-yl)benzaldehyde (480 mg, 1.249 mmol) was dissolved in DMSO (3000 μl) and hydrazine (118 μl, 3.75 mmol) was added. The reaction was stirred at 100 °C for 1 day. LCMS showed complete conversion. The resulting solution was diluted with water and was extracted with EtOAc (3 × 40 ml). The combined organic layers were washed with brine, dried over magnesium sulfate, and concentrated. After the removal of solvent, the residue was re-dissolved in DMSO and was purified by prep-HPLC (eluting acetonitrile/water gradient with TFA modifier, linear gradient) to afford the title compound (Compound 1) as a solid. LCMS: 384 $[M+H]^+$

Compound 1 was subjected to chiral SFC separation under the following conditions and four isomers were obtained.

Column & dimensions: Phenomenex, Lux (250 mm × 21 mm × 4 um)
Outlet Pressure (bar): 100
UV wavelength (nm): 220
Flow rate (ml/min): 70
Modifier: Methanol + 0.25% Dimethyl Ethyl Amine
% modifier in $CO_2$: 40
Sample amount (mg): 400
Diluent: Methanol/ACN
Diluent volume (mL): 25
Injection volume (mL): 0.4

Peak 1 Rf = 3.14 min: isomer 1 (Compound 1-1): $^1H$ NMR (400 MHz, DMSO-d) δ ppm 13.04 (br, 2H), 10.77 (s, 1H), 8.50 (s, 1H), 8.13 (s, 1H), 7.20 (s, 1H), 6.50 (s, 1H), 3.76 (d, J = 8.5 Hz, 1H), J = 3.59 (dd, J = 14 Hz, 4 Hz, 1H), 3.04 (t, J = 14.1 Hz, 1H), 2.76 (t, J = 8.5 Hz, 1H), 2.18 (m, 1H), 2.08 (m, 1H), 1.73 (d, J = 8.5 Hz, 1H), 0.76 (d, J = 8.4 Hz, 3H).

Peak 2 Rf = 4.02 min: isomer 2 (Compound 1-2): $^1H$ NMR (400 MHz, DMSO-d) δ ppm 13.04 (br, 2H), 10.59 (br, 1H), 8.13 (s, 1H), 7.90 (s, 1H), 7.17 (s, 1H), 6.50 (s, 1H), 3.72 (d, J = 8.5 Hz, 1H), J = 3.63 (d, J = 14 Hz, 1H), 3.45 (t, J = 12.1 Hz, 1H), 3.25 (t, J = 12 Hz, 1H), 2.02 (m, 1H), 1.95 (m, 1H), 1.13 (m, 1H), 0.89 (d, J = 8.4 Hz, 3H).

Peak 3 Rf = 5.15 min: isomer 3 (Compound 1-3): $^1$H NMR (400 MHz, DMSO-d) δ ppm 13.04 (br, 2H), 10.59 (br, 1H), 8.13 (s, 1H), 7.90 (s, 1H), 7.17 (s, 1H), 6.50 (s, 1H), 3.72 (d, J = 8.5 Hz, 1H), J = 3.63 (d, J = 14 Hz, 1H), 3.45 (t, J = 12.1 Hz, 1H), 3.25 (t, J = 12 Hz, 1H), 2.02 (m, 1H), 1.95 (m, 1H), 1.13 (m, 1H), 0.89 (d, J = 8.4 Hz, 3H).

Peak 4 Rf = 6.58 min: isomer 4 (Compound 1-4): $^1$H NMR (400 MHz, DMSO-d) δ ppm 13.04 (br, 2H), 10.77 (s, 1H), 8.50 (s, 1H), 7.20 (s, 1H), 6.50 (s, 1H), 3.76 (d, J = 8.5 Hz, 1H), J = 3.59 (dd, J = 14 Hz, 4 Hz, 1H), 3.04 (t, J = 14.1 Hz, 1H), 2.76 (t, J = 8.5 Hz, 1H), 2.18 (m, 1H), 2.08 (m, 1H), 1.73 (d, J = 8.5 Hz, 1H), 0.76 (d, J = 8.4 Hz, 3H).

An alternative process for the synthesis of Compound 1-1, a specific stereoisomer of Compound 1, is provided below.

**Alternative process for synthyzing 1-1 ((SS,6R)-8-(6-Bromo-1H-indazol-4-yl)-6-methyl-1,3,8-triazaspiro[4.5]decane-2,4-dione) (Compound 1-1)**

Step 1. (R)-1-Benzyl-3-methylpiperidin-4-one (2S,3S)-2,3-bis((4-methylbenzoyl)oxy)succinate

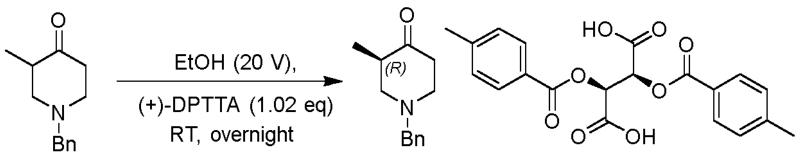


I-1

Into a 20 L flask equipment with an overhead stirring bar was added a solution of (2S,3S)-2,3-bis((4-methylbenzoyl)oxy)succinic acid (775 g, 2007 mmol) in EtOH (4000 ml). To the resulting solution was added a solution of 1-benzyl-3-methylpiperidin-4-one (400 g, 1968 mmol) in EtOH (2000 ml) dropwise at 30 °C. The resulting solution was stirred overnight. A sample was taken and chiral HPLC showed ee% of the resulting solid is 90%. The solid was filtrated out to afford the title compound as a solid with 90% ee.

Step 2. (5S,6R)-8-Benzyl-6-methyl-1,3,8-triazaspiro[4.5]decane-2,4-dione

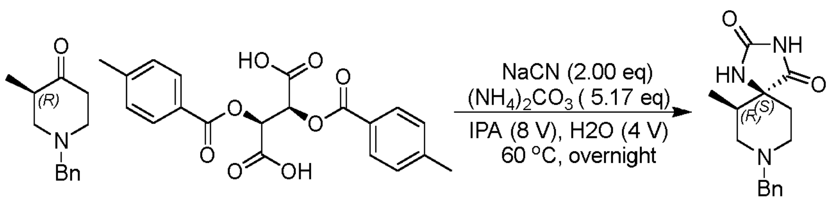


I-1 I-2

Into a 20L flask equiped with a reflux condenser was added 2-propanol (5360 ml) and water (2680 ml). Then $(NH_4)_2CO_3$ (564 g, 5875 mmol) was added to the solvent and the resulting mixture was stirred for 30 min to dissolve all staring material. To a 22 °C solution was added (R)-1-benzyl-3-methylpiperidin-4-one (2S,3S)-2,3-bis((4-methylbenzoyl)oxy)succinate (670 g, 1136 mmol) and this was followed by the addition of sodium cyanide (111 g, 2273 mmol) for 10 min. The resulting solution was heated to 60 °C overnight. LCMS showed the reaction was complete. The resulting solution was cooled to RT and water (8040 ml) was added dropwise. The resulting solution was stirred overnight. A solid was filtrated and dried in an oven. This process resulted in 240 g of a crude material. To the crude material was added 4800 ml of acetone overnight resulting in a slurry. A solid was filtrated and the title compound was obtained as a solid with 100% ee.

Step 3. (5S,6R)-6-Methyl-1,3,8-triazaspiro[4.5]decane-2,4-dione

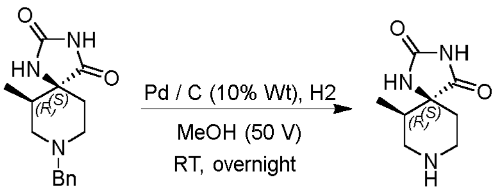


I-2 I-3

Into a 2L round bottle was added MeOH (1500 ml) and this was followed by the addtion of (5S,6R)-8-benzyl-6-methyl-1,3,8-triazaspiro[4.5]decane-2,4-dione (30 g, 110 mmol). The resulting solution was stirred for 15 min to dissolve all solid. Pd-C (3.5 g, 32.9 mmol) 10%wt was added to the resulting solution. The resulting solution was degassed, filled with nitrogen gas 3 times, and then charged with hydrogen balloon. The resulting solution was stirred at 30 °C for 3 h, cooled to RT, and was flushed with nitrogen. The solution was concentrated directly to afford the title compound.

Step 4. 4-Bromo-2-fluoro-6-((5S,6R)-6-methyl-2,4-dioxo-1,3,8-triazaspiro[4.5]decan-8-yl)benzaldehyde

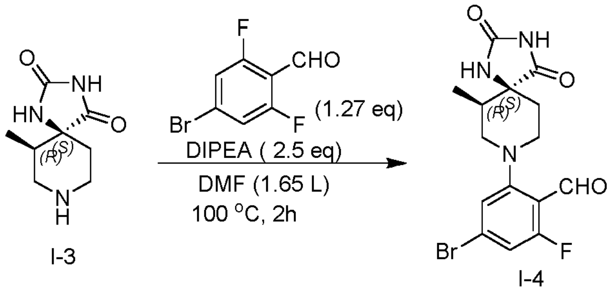


I-3 I-4

Into a flask equiped with a reflux condenser was added a solution of (5S,6R)-6-methyl-1,3,8-triazaspiro[4.5]decane-2,4-dione (165 g, 720 mmol) in DMF (1650 ml). To the solution was added DIPEA (315 ml, 1801 mmol) and 4-bromo-2,6-difluorobenzaldehyde (202 g, 915 mmol) in sequence. The resulting solution was heated to 100 °C for 2h. LCMS showed the starting material was consumed. The resulting solution was cooled to RT. Pd/C from last step was filtrated out through a celite pad. The filtrate was diluted by adding water (4950 ml) dropwise. The resulting solution was stirred overnight. The solid was filtrated and washed with water. The solid was dried in an oven. A slurry was formed by adding heptane (3300 ml) to the resulting solid for 2 h at RT. The resulting solid was dissolved in THF (3300 ml) and thiol silica gel (PSA-10, 25g ) was added. The resulting solution was stirred at 30 °C overnight. The silica gel was removed by filtration resulting in the title compound as a solid.

Step 5. (5S,6R)-8-(6-Bromo-1H-indazol-4-yl)-6-methyl-1,3,8-triazaspiro[4.5]decane-2,4-dione

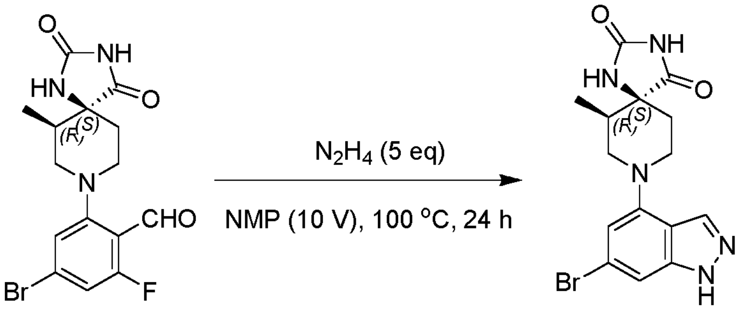


I-4 Compound 1-1

Into a flask equiped with a reflux condenser was placed a solution of 4-bromo-2-fluoro-6-((5S, 6R)-6-methyl-2,4-dioxo-1,3,8-triazaspiro[4.5]decan-8-yl)benzaldehyde (240 g, 625 mmol) in NMP (2400 ml). $N_2H_4.H_2O$ (195 g, 3123 mmol) was added to the solution at RT. The resulting solution was heated to 100 °C for 24 h. LCMS showed the reaction was

complete. The solution was cooled to RT and filtrated to remove the Pd/C precipitate from the solvent. The filtrate was diluted by adding water (1.20E+04 ml) dropwise. The solid was filtrated and washed with 5L water 4 times. The solid was dried in an oven under vacuum at 60 °C for 8 h to afford 180g of a crude which was washed with acetone (900 ml) to afford 155g of a solid (97% purity). The solid was stirred as a slurry in 755 ml acetone at 50 °C for 2 h. The solution was cooled to RT and 775 ml heptane was added dropwise. The resulting mixture was stirred overnight and filtrated. The solid was dried under nitrogen flow to afford the title compound.

LC-MS: (ES, *m/z):* 378 $[M-1]^-$
H-NMR: (300 MHz, DMSO-$d_6$, *ppm):* δ 13.24 - 12.95 (m, 1H), 10.77 (s, 1H), 8.49 (s, 1H), 8.15 (s, 1H), 7.20 (s, 1H), 6.48 (d, J = 1.4 Hz, 1H), 3.74 (d, J = 12.7 Hz, 1H), 3.57 (dd, J = 13.1, 3.9 Hz, 1H), 3.02 (td, J = 13.1, 2.5 Hz, 1H), 2.75 (t, J = 12.4 Hz, 1H), 2.26 - 1.98 (m, 2H), 1.79 - 1.65 (m, 1H), 0.74 (d, J = 6.6 Hz, 3H).

Biological Assays

IDO1 Enzymatic Assay (A)

Compounds to be tested were serially diluted in ten 3-fold steps in DMSO starting from 10 mM DMSO stocks. Compound dilutions or DMSO alone were then dispensed from the dilution plate into a Greiner black 384-well assay plate (catalog #781086) using an Echo 555 acoustic liquid handler (Labcyte). HIS-tagged IDO1 protein (Genbank #: NM_002164) was recombinantly expressed in Escherichia coli using ZYP5052 auto induction media supplemented with 500 μM delta aminolevulinic acid for 48 h at 16 °C. IDO1 protein was purified using $Ni^{2+}$-affinity resin and size exclusion chromatography. Purified protein was then diluted in assay buffer (50 mM Tris pH 7.0, 1% glycerol, 20 μM methylene blue, 0.05% Tween-20, 20 mM sodium ascorbate, 100 units/mL catalase) to obtain a final IDO1 concentration of 40 nM. IDO1 solution (30 μM) or buffer alone (30 μM) were dispensed to wells of the assay plate using a BioRAPTR liquid dispenser (Beckman Coulter). Assay plates containing compound and IDO1 enzyme were incubated at RT for 30 min. Afterwards, 10 μL of 400 μM tryptophan in assay buffer were added to each well of the assay plate using a BioRAPTR liquid dispenser. Plates were incubated at RT for 60 min and reactions were quenched by addition of 10 μL of 0.5 M methyl isonipecotate in dimethyl sulfoxide. Plates were sealed and incubated at 37 °C for 4 h or 50 °C for 2 h. The plates were allowed to cool and then centrifuged for 1 min at 1000xg. The resulting fluoresence was measured in an Envision plate reader (Perkin Elmer) with a 400/25 nm excitation filter and an 510/20 nm emission filter. The fluoresence intensity of each well was corrected for the background observed in wells that did not receive IDO1 and was expressed as a fraction of the intensity observed in wells that received **IDO1** enzyme and DMSO only. Potencies were calculated by linear least squares fit to the four parameter logistic IC50 equation.

IDO1 Cellular Assay in HeLa Cells Stimulated with IFNγ (B)

HeLa cells were cultured in complete HeLa culture medium (90% EMEM, 10% heat-inactivated fetal bovine serum) and expanded to about $1x10^9$ cells. The cells were then collected and frozen down at $1x10^7$ cells/vial in 1 mL frozen medium (90% complete HeLa culture medium, 10% DMSO).
Compounds to be tested were serially diluted in ten 3-fold steps in DMSO starting from 10 mM DMSO stocks in Echo low volume plate(s). Compound dilutions or DMSO alone were then dispensed from the dilution plate(s) into Greiner black 384-well assay plate(s) (catalog #781086, 50 nL/well) using an Echo 550 acoustic liquid handler (Labcyte).
Frozen HeLa cells were thawed and transferred into HeLa assay medium (99% complete HeLa culture medium, 1% Pen/Strep) with 20 mL medium/vial of cells. The cells were spun down at 250g in a table top centrifuge for 5 min and suspended in the same volume of HeLa assay medium. The cells were then counted and adjusted to a density of $2 \times 10^5$ cells/mL in HeLa assay medium. Sterile L-tryptophan were added to the cells with the final concentration of 300 uM L-tryptophan. A small aliquot (2 mL/plate) of HeLa cells was set aside and was not treated with IFNγ, to serve as the Max-E control. The rest of HeLa cells were added with sterile IFNγ (Cat# 285-IF, R & D systems) with a final concentration of 100 ng/mL.
HeLa cells with and without IFNγ were dispensed to the respective wells of 384-well assay plates containing the compounds. The plates were incubated for about 48 h in a 37 °C, 5% $CO_2$ incubator. Afterwards, 12 μL of 0.5 M methyl isonipecotate in dimethyl sulfoxide was added into each well and the plates were sealed and incubated at 37 °C without $CO_2$ overnight. The plates were centrifuged for 1 min at 200xg. The resulting fluorescence was measured in a Spectramax plate reader (Molecular Devices) with a 400 nm excitation filter and a 510 nm emission filter.
The fluorescence intensity of each well was corrected for the background observed in wells with non-IFNγ-treated cells and was expressed as a fraction of the intensity observed in wells of IFNγ-treated cells and DMSO only. Potencies were calculated by linear least squares fit to the four parameter logistic $IC_{50}$ equation.
The biological activity data using the IDO1 cellular assay described above are summarized in the table below.

Compounds disclosed herein generally have $IC_{50}$ of about 1 nM to about 10,000 nM, or in specific embodiments, about 10 nM to about 1,000 nM, or in further specific embodiments, about 20 nM to about 800 nM, or in further specific embodiments, about 30 nM to about 500 nM, or in further specific embodiments, about 50 nM to about 400 nM. Specific $IC_{50}$ activity data for the exemplified compounds disclosed herein is provided in the following table.

TDO Cellular Assay (C)

Compounds were serially diluted in DMSO and acoustically dispensed (LabCyte Echo) into each well of a black 384-well cell culture plate (Greiner). SW48 cells were diluted to a density of $2\times10^5$ cells/ml in complete RPMI culture medium supplemented with 500 mM L-tryptophan and 50 mL SW48 cells were dispensed to each well of 384-well plates with a compound. Plates are gently shaken and then incubated for 48 hours at 37 °C in an incubator with 5% $CO_2$. After incubation, 12μl of the detection agent (0.5 M isonipecotate in DMSO) was added to each well and incubated at 37 °C overnight. The fluorescent signal was read on a Spectramax plate reader with excitation at 400 nm and emission at 510 nm. The percent inhibition was determined by normalization to wells containing no compound relative to wells receiving no cells. These data were fit to a four parameter logisitic equation to determine the $IC_{50}$.

IDO1 Human Whole Blood Assay (D)

Compounds to be tested were serially diluted in ten 3-fold steps in DMSO starting from 10 mM. 3 μL of compound dilutions or DMSO alone were then dispensed from the dilution plate into a polypropylene 96-well assay plate containing 97 μL of RPMI using an Echo 555 acoustic liquid handler (Labcyte). LPS and IFNγ was prepared in RPMI to a 10X of final conc. (1000 ng/mL), final concentration is 100 ng/mL.

Human whole blood was drawn in sodium heparin coated tubes from healthy internal donors. 240 μL of blood was transferred to each of the wells of a v-bottom 96 well plate. 30 μL of a compound was transferred from intermediate dilution plate, and incubated for 15 min. 30 μL of the LPS and IFNγ solution prepared above was then transferred to a blood sample and mixed thoroughly. Plate was covered with breathable membrane and incubated at 37 °C for overnight (18 h).

On day 2 isotope labeled standard of kynurenine and tryptophan was made in water at 10x concentration and 30 μL was added to the blood sample at 3 μM final concentration. The assay plates were centrifuged at 300xG for 10 min with no brake to separate plasma from red blood cells. 60 μL of plasma samples was removed without disturbing red blood cells. Plasma was diluted with RPMI in 1:1 ratio and proteins were precipitated out with two volumes of acetonitrile. The plates were centrifuged at 4000xG for 60 min. 20 μL of supernatant was carefully transferred to a 384 well plate contain 40 μL of 0.1% formic acid in water and analyzed by LC/MS/MS.

LC/MS/MS analyses were performed using Thermo Fisher's LX4-TSQ Quantum Ultra system. This system consists of four Agilent binary high-performance liquid chromatography (HPLC) pumps and a TSQ Quantum Ultra triple quadrupole MS/MS instrument. For each sample, 5 μL were injected onto an Atlantis T3 column (2.1 mm x 150 mm, 3 μm particle size) from Waters. The mobile phase gradient pumped at 0.8 mL/min was used to elute the analytes from the column at 25 °C. The elution started at 0% B increasing linearly to 25% B at 6.5 min, holding at 25% for 1 min, re-equilibrating to 10 min. Mobile phase A consisted of 0.1% formic acid in water. Mobile phase B consisted of 0.1% of formic acid in acetonitrile. Data was acquired in positive mode using a HESI interface. The operational parameters for the TSQ Quantum Ultra instrument were a spray voltage of 4000 V, capillary temperature of 380 °C, vaporizer temperature 400 °C, shealth gas 60 arbitrary units, Aux gas 20 arbitrary units, tube lens 85 and collision gas 1.2 mTorr. SRM chromatograms of kynurenine (Q1: 209.2>Q3:94.0) and internal standard (Q1: 215.3>Q3:98.2) were collected for 90 sec. The peak area was integrated by Xcalibur Quan software. The ratios between the kynurenine generated in the reaction and 2D6-Kynurenine spiked-in internal standard were used to generate percentage inhibition and $IC_{50}$ values. Compounds were titrated and $IC_{50}$ values were calculated by 4 parameter sigmoidal curve fitting formula.

The biological activity data of Compound 1-1 and comparative compound CC-1 using the biological assay methods A-D described above are summarized in the table below.

| Compound # | Structure | IDO enzymatic $IC_{50}$, nM (A) | IDO hela (B) /TDO SW48 cell $IC_{50}$, nM (C) | IDO WB $IC_{50}$, nM (D) |
|---|---|---|---|---|
| 1-1 | 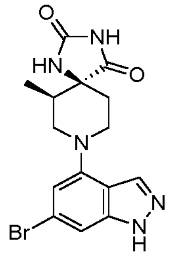 | 129 | 77/247 | 345 |
| CC-1 | 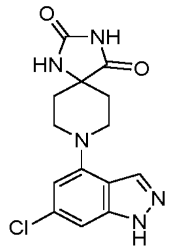 | 113 | 97/206 | 556 |

The activities of compound 1-1 and comparative compound CC-1 against off-target kinases are provided in the table below. The activity data was obtained in a panel of 265 and 264 for compounds 1-1 and CC-1, respectively, using the ZLYTE and ADAPTA assay methods indicated in the table below.

Estimated $IC_{50}$ (uM)

| Kinase | Compound 1-1 | Comparative CC-1 | Method |
|---|---|---|---|
| CLK2 | 0.73 | 0.35 | ZLYTE |
| RPS6KB1 | 1.9 | 1.7 | ZLYTE |
| FLT3(D835Y) | 2 | 1.5 | ZLYTE |
| CDK5/P25 | >2.0 | 1.2 | ZLYTE |
| SGK2 | >2.0 | 1.3 | ZLYTE |
| MELK | >2.0 | 1.4 | ZLYTE |
| CDK2 | >2.0 | 1.5 | ZLYTE |
| CDK5/P35 | >2.0 | 1.6 | ZLYTE |
| TYK2 | >2.0 | 2 | ZLYTE |
| CDK9 (Cyclin T1) | >2.0 | 0.15 | ADAPTA |
| LRRK2 (G2019S) | >2.0 | 0.94 | ADAPTA |

As can be seen from the above table, compound 1-1 is less active (i.e., exhibiting higher $IC_{50}$ values) against the listed off-target kinases than comparative compound CC-1. The instant compounds are potent IDO and TDO dual inhibitors while showing less off-target activities as compared to known compounds.

The ZLYTE anf ADAPTA assys are described below. More detailed assasy conditions and procedures are available on ThermoFisher's website, for eample, in the Technology Overview section of the following site: https://www.thermofisher.com/us/en/home/products-and-services/services/custom-services/screening-and-profiling-services/selectscreen-profiling-service/selectscreen-kinase-profiling-service.html.

ZLYTE Assay Method

The ZLYTE biochemical assay employs a fluorescence-based, coupled-enzyme format and is based on the differential sensitivity of phosphorylated and non-phosphorylated peptides to proteolytic cleavage. The peptide substrate is labeled with two fluorophores-one at each end-that make up a Fluorescence Resonance Energy Transfer (FRET) pair.

In the primary reaction, the kinase transfers the gamma-phosphate of ATP to a single tyrosine, serine or threonine residue in a synthetic FRET-peptide. In the secondary reaction, a site-specific protease recognizes and cleaves non-phosphorylated FRET-peptides. Phosphorylation of FRET-peptides suppresses cleavage by the Development Reagent. Cleavage disrupts FRET between the donor (i.e., coumarin) and acceptor (i.e., fluorescein) fluorophores on the FRET-peptide, whereas uncleaved, phosphorylated FRET-peptides maintain FRET. A ratiometric method, which calculates the ratio (the Emission Ratio) of donor emission to acceptor emission after excitation of the donor fluorophore at 400 nm, is used to quantitate reaction progress, as shown in the equation below.

$$\text{Emission Ratio} = \text{Coumarin Emission (445 nm)} / \text{Fluorescein Emission (520 nm)}$$

Both cleaved and uncleaved FRET-peptides contribute to the fluorescence signals and therefore to the Emission Ratio. The extent of phosphorylation of the FRET-peptide can be calculated from the Emission Ratio. The Emission Ratio will remain low if the FRET-peptide is phosphorylated (i.e., no kinase inhibition) and will be high if the FRET-peptide is non-phosphorylated (i.e., kinase inhibition).

Test Compounds

The test compounds are screened in 1% DMSO (final) in the well. For 10 point titrations, 3-fold serial dilutions are conducted from the starting concentration.

Peptide/Kinase Mixtures

All peptide/kinase mixtures are diluted to a 2X working concentration in the appropriate kinase buffer.

ATP Solution

All ATP solutions are diluted to a 4X working concentration in kinase buffer (50 mM HEPES pH 7.5, 0.01% BRIJ-35, 10 mM MgCl2, 1 mM EGTA).

Development Reagent Solution

The development reagent is diluted in development buffer.

10X Novel PKC Lipid Mix:

2 mg/ml Phosphatidyl serine, 0.2 mg/ml DAG in 20 mM HEPES, pH 7.4, 0.3% CHAPS

For 5 mL 10X Novel PKC Lipid Mix:

1. Add 10 mgs Phosphatidyl Serine and 1 mg DAG (Avanti Polar Lipids Part# 800811C) to a glass tube.
2. Remove the chloroform from lipid mixture by evaporating to a clear, thin film under a stream of nitrogen. Continuous rotation of the tube, at an angle to ensure maximum surface area of the lipid solution, will promote the thinnest film.
3. Add 5 mL resuspension buffer, 20 mM HEPES, 0.3% CHAPS, pH 7.4, to the dried lipid mix.
4. Heat gently to 50-60 °C for 1-2 minutes and vortex in short intervals until the lipids are dissolved to a clear or slightly hazy solution. The lipids are typically in solution after 2-3 heat/vortex cycles.
5. Cool to room temperature, aliquot into single use volumes and store at -20 °C.

Assay Protocol

Bar-coded Corning, low volume NBS and black 384-well plate can be used following the protocol below.

1. 100 nL - 100X Test Compound in 100% DMSO;
2. 2.4 μL - kinase buffer;
3. 5 μL - 2X peptide/kinase mixture;
4. 2.5 μL - 4X ATP solution;
5. 30-second plate shake;
6. 60-minute kinase reaction incubation at room temperature;
7. 5 μL - development reagent solution;
8. 30-second plate shake;
9. 60-minute development reaction incubation at room temperature; and
10. Read on fluorescence plate reader and analyze the data.

ADAPTA Assay

The Adapta universal kinase assay is a homogenous, fluorescent based immunoassay for the detection of ADP. In contrast to ATP depletion assays, the Adapta assay is highly sensitive to ADP formation such that a majority of the signal change occurs in the first 10-20% conversion of ATP to ADP. This makes the Adapta universal kinase assay well suited for use with low activity kinases.

The principle of the Adapta universal kinase assay is outlined below. The assay itself can be divided into two phases: a kinase reaction phase, and an ADP detection phase. In the kinase reaction phase, all components required for the kinase reaction are added to the well, and the reaction is allowed to incubate for 60 minutes. After the reaction, a detection solution consisting of a europium labeled anti-ADP antibody, an Alexa Fluor 647 labeled ADP tracer, and EDTA (to stop the kinase reaction) is added to the assay well. ADP formed by the kinase reaction (in the absence of an inhibitor) will displace the Alexa Fluor 647 labeled ADP tracer from the antibody, resulting in a decrease in the TR-FRET signal. In the presence of an inhibitor, the amount of ADP formed by the kinase reaction is reduced, and the resulting intact antibody tracer interaction results in a high TR-FRET signal.

ADP formation is determined by calculating the emission ratio from the assay well. The emission ratio is calculated by dividing the intensity of the tracer (acceptor) emission by the intensity of the Eu (donor) emission at 615 nm as shown in the equation below.

$$\text{Emission Ratio} = \text{AlexaFluor647 Emission (665 nm)/Europium Emission (615 nm)}$$

Since the Adapta technology measures ADP formation (i.e. conversion of ATP to ADP) it can be used to measure any type of ATP hydrolysis, including intrinsic ATPase activity of kinases. In this case, the substrate is water, not a lipid or peptide. The SelectScreen service screens CHUK in this way, so a substrate is not included in the kinase reaction.

Adapta Assay Conditions

Test Compounds

The test compounds are screened in 1% DMSO (final) in the well. For 10 point titrations, 3-fold serial dilutions are conducted from the starting concentration.

Substrate/Kinase Mixtures

All substrate/kinase mixtures are diluted to a 2X working concentration in the appropriate kinase buffer.

ATP Solution

All ATP solutions are diluted to a 4X working concentration in water.

Detection Mix

The detection mix is prepared in TR-FRET dilution buffer. The detection mix consists of EDTA (30 mM), Eu-anti-ADP antibody (6 nM) and ADP tracer. The detection mix contains the EC60 concentration of tracer for 5-150 μM ATP.

Assay Protocol

Bar-coded Corning, low volume, and white 384-well plate can be used following the protocol below.

1. 100 nL - 100X test compound in 100% DMSO;
2. 2.4 μL - 30 mM HEPES;
3. 2.5 μL - 4X ATP Solution;
4. 5 μL - 2X substrate/kinase mixture;
5. 30-second plate shake;
6. 1-minute centrifuge at 1000 x g;
7. 60-minute kinase reaction incubation at room temperature;
8. 5 μL - detection mix;
9. 30-second plate shake;
10. 1-minute centrifuge at 1000 x g;
11. 60-minute detection mix equilibration at room temperature; and
12. Read on fluorescence plate reader and analyze the data.

## Claims

**1.** A compound of formula:

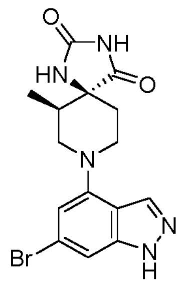
,

or a pharmaceutically acceptable salt thereof.

**2.** A pharmaceutical composition which comprises a pharmaceutically acceptable carrier and a compound of claim 1, or a pharmaceutically acceptable salt thereof.

**3.** A compound of claim 1, or a pharmaceutically acceptable salt thereof for use in treating cancer, viral infection, HCV infection, depression, neurodegenerative disorders, trauma, age-related cataracts, organ transplantation, or autoimmune disease.

**4.** The compound or pharmaceutically acceptable salt for use of claim 3, wherein the cancer is selected from colon cancer, pancreas cancer, breast cancer, prostate cancer, lung cancer, brain cancer, ovary cancer, cervix cancer, testes cancer, renal cancer, head and neck cancer, lymphoma, leukemia, and melanoma.

**5.** A compound of claim 1, or a pharmaceutically acceptable salt thereof for use in therapy.

**6.** A combination comprising a compound of claim 1, or a pharmaceutically acceptable salt thereof and one or more other active agents.

## Patentansprüche

**1.** Eine Verbindung der Formel:

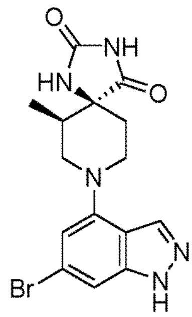

oder ein pharmazeutisch annehmbares Salz davon.

2. Eine pharmazeutische Zusammensetzung, die einen pharmazeutisch annehmbaren Träger und eine Verbindung nach Anspruch 1 oder ein pharmazeutisch annehmbares Salz davon umfasst.

3. Eine Verbindung nach Anspruch 1 oder ein pharmazeutisch annehmbares Salz davon zur Verwendung bei der Behandlung von Krebs, einer Virusinfektion, einer HCV-Infektion, Depression, neurodegenerativen Störungen, Trauma, altersbedingten Katarakten, Organtransplantation oder einer Autoimmunerkrankung.

4. Die Verbindung oder das pharmazeutisch annehmbare Salz zur Verwendung nach Anspruch 3, wobei der Krebs ausgewählt ist aus Kolonkarzinom, Bauchspeicheldrüsenkrebs, Brustkrebs, Prostatakarzinom, Lungenkrebs, Gehirntumor, Ovarialkarzinom, Zervixkarzinom, Hodenkrebs, Nierenkrebs, Kopf-Hals-Tumor, Lymphom, Leukämie und Melanom.

5. Eine Verbindung nach Anspruch 1 oder ein pharmazeutisch annehmbares Salz davon zur Verwendung in der Therapie.

6. Eine Kombination, die eine Verbindung nach Anspruch 1 oder ein pharmazeutisch annehmbares Salz davon und einen oder mehrere andere Wirkstoffe umfasst.

## Revendications

1. Composé de formule :

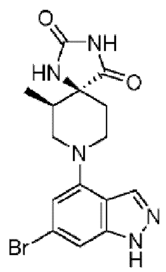,

ou un sel pharmaceutiquement acceptable de celui-ci.

2. Composition pharmaceutique comprenant un support pharmaceutiquement acceptable et un composé selon la revendication 1, ou un sel pharmaceutiquement acceptable de celui-ci.

3. Composé selon la revendication 1, ou un sel pharmaceutiquement acceptable de celui-ci, pour utilisation dans le traitement d'un cancer, d'une infection virale, d'une infection par le VHC, d'une dépression, de troubles neurodégénératifs, de traumatismes, de cataractes liées à l'âge, de transplantations d'organes ou de maladies autoimmunes.

4. Composé ou un sel pharmaceutiquement acceptable de celui-ci pour utilisation selon la revendication 3, dans lequel

le cancer est choisi parmi un cancer du côlon, un cancer du pancréas, un cancer du sein, un cancer de la prostate, un cancer du poumon, un cancer du cerveau, un cancer de l'ovaire, un cancer du col de l'utérus, un cancer des testicules, un cancer du rein, un cancer de la tête et du cou, un lymphome, une leucémie et un mélanome.

**5.** Composé selon la revendication 1, ou un sel pharmaceutiquement acceptable de celui-ci, pour utilisation en thérapie.

**6.** Combinaison comprenant un composé selon la revendication 1, ou un sel pharmaceutiquement acceptable de celui-ci, et un ou plusieurs autres agents actifs.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016071293 A
- WO 01002369 A
- WO 02068470 A
- WO 02010192 A
- US 7488802 B
- US 7521051 B
- US 8008449 B
- US 8354509 B
- US 8168757 B
- WO 2004004771 A
- WO 2004072286 A
- WO 2004056875 A
- US 20110271358 A
- WO 2013019906 A
- WO 2010077634 A1
- US 8383796 B
- WO 2010027827 A
- WO 2011066342 A


### Non-patent literature cited in the description


- **LOGAN et al.** *Immunology*, 2002, vol. 105, 478-87
- **BROWN et al.** *Adv. Exp. Med. Biol*, 1991, vol. 294, 425-35
- **PORTULA et al.** *Blood*, 2005, vol. 106, 2382-90
- **MEDAWAR**. *Symp. Soc. Exp. Biol.*, 1953, vol. 7, 320-38
- **MOAN et al.** *Science*, 1998, vol. 281, 1191-3
- **UYTTENHOVE et al.** *Nature Med.*, 2003, vol. 9, 1269-74
- **MULLER et al.** *Nature Med.*, 2005, vol. 11, 312-9
- **MUNN et al.** *Science*, 2002, vol. 297, 1867-70
- **MUNN et al.** *J. Clin. Invest*, 2004, vol. 114 (2), 280-90
- **GROHMANN et al.** *Trends Immunol*, 2003, vol. 24, 242-8
- **WIRLEITNER et al.** *Curr. Med. Chem.*, 2003, vol. 10, 1581-91
- **SCHMIDT SK et al.** *Eur J Immunol.*, 2009, vol. 39, 2755-64
- **MICHELS H et al.** *Sci Rep.*, 2016, vol. 6, 39199
- **ELBERS F et al.** *Mediators Inflamm.*, 2016, 1638916
- **LIU X et al.** *Blood.*, 2010, vol. 115, 3520-30
- **ABDEL-MAGID AF.** *ACS Med Chem Lett.*, 2017, vol. 8, 11-3
- **PANTOURIS G et al.** *J Enzyme Inhib Med Chem.*, 2016, vol. 31, 70-8
- **CHEONG JE et al.** *Trends Pharmacol Sci.*, 2018, vol. 39, 307-25
- Remington's Pharmaceutical Sciences. Mack Publishing Company
- The Handbook of Pharmaceutical Additives. Gower Publishing Limited
- The Handbook of Pharmaceutical Excipients. the American Pharmaceutical Association and the Pharmaceutical Press
- **T. W. GREENE** ; **P. G. M. WUTS**. Protective Groups in Organic Synthesis. Wiley, 1999